# EUROPEAN PATENT APPLICATION

(11) **EP 3 305 903 A1**
(43) Date of publication of application: **11.04.2018**
(21) Application number: 16002120.0
(22) Date of filing: 04.10.2016
(51) Int. Cl.: C12N 15/67, C12N 15/11

(54) **RNA TOOLBOX**

(71) Applicant: Rheinisch-Westfälische Technische Hochschule (RWTH) Aachen, 52056 Aachen (DE)
(72) Inventor: Thalmann, Beat, 69115 Heidelberg (DE)
(74) Representative: Müller-Boré & Partner Patentanwälte PartG mbB

(57) **Abstract**

The present invention relates to isolated nucleic acids comprising nucleic acid sequences derived from the *Cricetulus griseus* small nucleolar RNAs (snoRNAs; Snords) Snord74 (cS74) and Snord78 (cS78), to vectors and host cells comprising the same, and methods of protecting and/or stabilizing an RNA of interest in a host cells, expressing a protein of interest in a host cell, and/or increasing the cell density and/or cell viability and/or productivity in a culture of host cells, said methods using the nucleic acids of the present invention.

## Description

The present invention relates to isolated nucleic acids comprising nucleic acid sequences derived from the *Cricetulus griseus* small nucleolar RNAs (snoRNAs; Snords) Snord74 (cS74) and Snord78 (cS78), to vectors and host cells comprising the same, and methods of protecting and/or stabilizing an RNA of interest in a host cells, expressing a protein of interest in a host cell, and/or increasing the cell density and/or cell viability and/or productivity in a culture of host cells, said methods using the nucleic acids of the present invention.

Current systems for the production of proteins rely on the overexpression, downregulation and/or disruption of suitable target genes, which need to be identified beforehand. However, a major limiting factor in the production of proteins such as therapeutic proteins or other proteinaceous biotechnological products is the low stability of the mRNA encoding said proteins. The positive correlation between the retention time of an mRNA and the amount of protein expressed form said mRNA has been shown in numerous publications.

However, means for specifically modulating the retention time of mRNA or of other RNA species such as ribosomal RNA (rRNA), small nuclear RNA (snRNA), and other non-coding RNAs (ncRNAs) in a targeted manner have so far been elusive. Such means could allow for e.g. the targeted stabilization of such RNA species, an improved maturation of rRNA, or improved splicing, maturation, stability and/or translation of mRNA via specific protection and/or improved maturation of snRNAs.

Small nucleolar RNAs are a class of small RNA molecules that primarily guide chemical modifications of other RNAs. There are two main classes of snoRNAs, *i.e.,* C/D box snoRNAs which are associated with methylation of target RNAs, and H/ACA box snoRNAs which are associated with pseudouridylation. Although snoRNAs have so far not been used for the targeted stabilization of arbitrary RNA species of interest, they might form a basis for achieving the goals formulated above.

Therefore, the technical problem underlying the present invention is the provision of means for the specific and targeted stabilization of arbitrarily selected RNA species of interest.

The solution to the above technical problem is achieved by the embodiments characterized in the claims.

In particular, in a first aspect, the present invention relates to an isolated nucleic acid, comprising at least one of the following structures (I) and (II): 5′ SEQ ID NO: 1 Sequence A SEQ ID NO: 3 Sequence B SEQ ID NO: 5 3′ (I) 
5′
SEQ ID NO: 7
Sequence C
SEQ ID NO:
9 or 51 or 56
Sequence D
CTGRAGAA
3′
(II)
 wherein Sequence A, Sequence B, Sequence C, and Sequence D are independently nucleic acid sequences that (i) have a length of 7 to 25 base pairs (bp), preferably 9 to 18 bp, more preferably 11 to 14 bp, and (ii) are complementary to either the same or to two different RNAs of interest. Preferably, said sequences are complementary to the 5'-UTR (untranslated region) and/or the coding sequence (CDS) and/or the 3'-UTR of previously defined targets (such as mRNA, tRNA, rRNA, ncRNA, pre-miRNA, or snRNA targets). The target specific sequences A-B and C-D, respectively, are not complementary avoiding RNA interference or other interfering mechanisms by stem-loop formation. The distance between the complementary target sequences for specific sequences A-B and C-D, respectively, does beneficiary not possess the length of SEQ ID NOs: 3 and 9 or 51 or 56, respectively, *i.e.,* said distance is preferably less than 16 bp or less than 27 bp, respectively. The 5'- and 3'-ends of the native and/or synthetic constructs (I) and (II) need stem formation which is e.g. achieved by implementing proper restriction sites known in the art.

The nucleic acids of the present invention are derived from the coding sequences of the *Cricetulus griseus* C/D box snoRNAs Snord74 (cS74) and Snord78 (cS78). In particular, the nucleotide sequences according to SEQ ID NOs: 1, 3, and 5 represent 5', intermediate, and 3' sequence elements of the coding sequence of cS74, respectively. Further, the nucleotide sequences according to SEQ ID NOs: 7 and 9, 51 or 56, as well as the sequence CTGRAGAA, represent 5', intermediate, and 3' sequence elements of the coding sequence of cS78, respectively. In this context, in the above structure (II), either SEQ ID NO: 9 or SEQ ID NO: 51 or SEQ ID NO: 56, which differ by one nucleotide, can be used. Thus, in certain embodiments of the nucleic acids of the present invention according to the above structure (II), SEQ ID NO: 9 is used. However, in certain other embodiments of the nucleic acids of the present invention according to the above structure (II), SEQ ID NO: 51 is used. Moreover, in certain other embodiments of the nucleic acids of the present invention according to the above structure (II), SEQ ID NO: 56 is used. In the sequences described herein, R represents A or G, W represents A or T, Y represents T or C, and N represent A, G, C or T, in accordance with standard IUPAC nucleotide nomenclature. In the nucleotide sequence according to SEQ ID NO: 1, it is preferred that the nucleotides in positions 7 and 13 are A (SEQ ID NO: 2). In the nucleotide sequence according to SEQ ID NO: 3, it is preferred that the nucleotides in positions 4 and 16 are A, and the nucleotide in position 10 is T (SEQ ID NO: 4). In the nucleotide sequence according to SEQ ID NO: 5, it is preferred that the nucleotide in position 4 is A (SEQ ID NO: 6). In the nucleotide sequence according to SEQ ID NO: 7, it is preferred that the nucleotides in positions 6 and 12 are A (SEQ ID NO: 8). In the nucleotide sequence according to SEQ ID NO: 9, it is preferred that (i) the nucleotides in positions 4, 9, and 21 are A, and the nucleotides in positions 24, 25, and 27 are T, A, and A, respectively (SEQ ID NO: 10), or (ii) the nucleotides in positions 4, 9, and 21 are A, and the nucleotides in positions 24, 25, and 27 are A, T, and A, respectively (SEQ ID NO: 11). In the nucleotide sequence according to SEQ ID NO: 51, it is preferred that (i) the nucleotides in positions 4, 9, and 21 are A, and the nucleotides in positions 24, 25, and 27 are T, A, and A, respectively (SEQ ID NO: 52), or (ii) the nucleotides in positions 4, 9, and 21 are A, and the nucleotides in positions 24, 25, and 27 are A, T, and A, respectively (SEQ ID NO: 53). In the nucleotide sequence according to SEQ ID NO: 56, it is preferred that (i) the nucleotides in positions 4, 9, and 21 are A, and the nucleotides in positions 24, 25, and 27 are T, A, and A, respectively (SEQ ID NO: 57), or (ii) the nucleotides in positions 4, 9, and 21 are A, and the nucleotides in positions 24, 25, and 27 are A, T, and A, respectively (SEQ ID NO: 58). In the nucleotide sequence CTGRAGAA in the above structure (II), it is preferred that the nucleotide in position 4 is A (CTGAAGAA). Thus, the nucleotide sequence according to SEQ ID NOs: 2, 4, 6, 8, 10 and 11, 52 and 53, 57 and 58, and the nucleotide sequence CTGAAGAA are preferred embodiments of the nucleotide sequences according to SEQ ID NOs: 1, 3, 5, 7, 9, 51, 56, and CTGRAGAA, respectively.

The nucleotide sequences Sequence A and Sequence B, as well as Sequence C and Sequence D, represent target RNA-specific sequence elements that provide specificity of the nucleic acids of the present invention for one or more target RNAs. These sequence elements are referred to herein as specific sequences. In this context, said specific sequences are interchangeable in the specific constructs disclosed herein. By way of example, in a case where e.g. in structure (I) as defined above, Sequence A is SEQ ID NO: 12 and Sequence B is SEQ ID NO: 13 (cf. construct cS74, described below), it is also possible that Sequence A is SEQ ID NO: 13, and Sequence B is SEQ ID NO: 12. This is indicated hereinafter by the term "or *vice versa".* In particular, the above exemplary situation would be indicated by using the term "in structure (I) as defined above, Sequence A is SEQ ID NO: 12 and Sequence B is SEQ ID NO: 13, or *vice versa*".

Thus, the nucleic acids of the present invention code for synthetic C/D box snoRNAs that specifically bind to the target RNA of interest and mediate methylation of the target RNA in a directed manner. By selecting suitable specific sequences (Sequence A, Sequence B, Sequence C, and/or Sequence D), any target RNA of interest can be stabilized within the cell. The underlying mechanism of action is the directed 2'-O-methylation of the target RNA, a, therefore, facilitated RNA-protein interaction and/or RNA-RNA interaction towards target RNA coverage, leading to delayed and/or abolished subsequent target degradation.

In structures (I) and (II) as depicted herein, the various boxes represent nucleotide sequences, and the lines connecting said boxes indicate which nucleotide sequences are followed by which. By way of example, a respectively depicted hypothetical structure (III) 5′ — Sequence I — Sequence II — Sequence III — 3′ (III), wherein Sequence I is the nucleotide sequence AA, Sequence II is the nucleotide sequence CC, and Sequence III is the nucleotide sequence GG, would represent the nucleotide sequence AACCGG. The lines at the 5' and 3' ends of structures (I) and (II) as depicted herein indicate the fact that the respective structures may be, and usually are, flanked by additional sequences such as e.g. restriction sites, intronic sequences, regulatory sequences and/or coding sequences.

In a preferred embodiment, in structure (I) as defined above, Sequence A is SEQ ID NO: 12 and Sequence B is SEQ ID NO: 13 or *vice versa.* In the former configuration (Sequence A is SEQ ID NO: 12 and Sequence B is SEQ ID NO: 13), structure (I) as a whole has the nucleotide sequence of SEQ ID NO: 14, preferably the nucleotide sequence of SEQ ID NO: 15. Thus, in a preferred embodiment, structure (I) as defined above is SEQ ID NO: 14, preferably SEQ ID NO: 15. In this embodiment, the nucleic acid of the present invention comprises a coding sequence that is identical to the wildtype coding sequence of the *Cricetulus griseus* Snord74 (cS74). Expression of this coding sequence in cell culture has been found to lead to increased cell specific productivity, thus affording e.g. increased protein expression (cf. the Examples below).

In another preferred embodiment, in structure (II) as defined above, Sequence C is SEQ ID NO: 16 and Sequence D is SEQ ID NO: 17 or *vice versa.* In this embodiment, it is preferred that SEQ ID NO: 9 is used. In the former configuration (Sequence C is SEQ ID NO: 16 and Sequence D is SEQ ID NO: 17), structure (II) as a whole has the nucleotide sequence of SEQ ID NO: 20, preferably the nucleotide sequence of SEQ ID NO: 21. Thus, in a preferred embodiment, structure (II) as defined above is SEQ ID NO: 20, preferably SEQ ID NO: 21. Respective constructs are designated herein as cSL7U2. In this embodiment, the nucleic acid of the present invention comprises specific sequences that target Luc7 mRNA of CHO cells (SEQ ID NO: 16) and U2 snRNA of CHO cells (SEQ ID NO: 17). Expression of this coding sequence in cell culture has been found to lead to increased cell viability and cell density in the cell culture, thus affording e.g. increased protein expression (cf. the Examples below).

In another preferred embodiment, in structure (II) as defined above, Sequence C is SEQ ID NO: 18 and Sequence D is SEQ ID NO: 19 or *vice versa.* In this embodiment, it is preferred that SEQ ID NO: 9 is used. In the former configuration (Sequence C is SEQ ID NO: 18 and Sequence D is SEQ ID NO: 19), structure (II) as a whole has the nucleotide sequence of SEQ ID NO: 22, preferably the nucleotide sequence of SEQ ID NO: 23. Thus, in a preferred embodiment, structure (II) as defined above is SEQ ID NO: 22, preferably SEQ ID NO: 23. Respective constructs are designated herein as cS2818. In this embodiment, the nucleic acid of the present invention comprises specific sequences that target 28S rRNA of CHO cells (SEQ ID NO: 18) and 18S rRNA of CHO cells (SEQ ID NO: 19). Expression of this coding sequence in cell culture has been found to lead to increased protein expression (cf. the Examples below).

In another preferred embodiment, in structure (I) as defined above, Sequence C is SEQ ID NO: 24 and Sequence D is SEQ ID NO: 25 or *vice versa.* In the former configuration (Sequence C is SEQ ID NO: 24 and Sequence D is SEQ ID NO: 25), structure (I) as a whole has the nucleotide sequence of SEQ ID NO: 26, preferably the nucleotide sequence of SEQ ID NO: 27. Thus, in a preferred embodiment, structure (II) as defined above is SEQ ID NO: 26, preferably SEQ ID NO: 27. Respective constructs are designated herein as cSssA. In this embodiment, the nucleic acid of the present invention comprises specific sequences that target the mRNA of structure specific recognition protein 1 (Ssrp1) (SEQ ID NOs: 24 and 25). Expression of this coding sequence in cell culture has been found to lead to increased protein expression (cf. the Examples below).

In another preferred embodiment, in structure (II) as defined above, Sequence C is SEQ ID NO: 28 and Sequence D is SEQ ID NO: 29 or *vice versa.* In this embodiment, it is preferred that SEQ ID NO: 9 is used. In the former configuration (Sequence C is SEQ ID NO: 28 and Sequence D is SEQ ID NO: 29), structure (II) as a whole has the nucleotide sequence of SEQ ID NO: 30, preferably the nucleotide sequence of SEQ ID NO: 31. Thus, in a preferred embodiment, structure (II) as defined above is SEQ ID NO: 30, preferably SEQ ID NO: 31. Respective constructs are designated herein as cSgIA. In this embodiment, the nucleic acid of the present invention comprises specific sequences that target the SV40 polyA signal sequence (SEQ ID NOs: 28 and 29). Expression of this coding sequence in cell culture has been found to lead to increased protein expression and/or titer (g/L) of the POI and/or productivity in a specific (pg/cell/d) as well as volumetric (g/L/h) manner (cf. the Examples below).

In another preferred embodiment, in structure (II) as defined above, Sequence C is SEQ ID NO: 32 and Sequence D is SEQ ID NO: 33 or *vice versa.* In this embodiment, it is preferred that SEQ ID NO: 9 is used. In the former configuration (Sequence C is SEQ ID NO: 32 and Sequence D is SEQ ID NO: 33), structure (II) as a whole has the nucleotide sequence of SEQ ID NO: 34, preferably the nucleotide sequence of SEQ ID NO: 35. Thus, in a preferred embodiment, structure (II) as defined above is SEQ ID NO: 34, preferably SEQ ID NO: 35. Respective constructs are designated herein as cSgIA. In this embodiment, the nucleic acid of the present invention comprises specific sequences that target the BGH (bovine growth hormone) polyA signal sequence (SEQ ID NOs: 32 and 33). Expression of this coding sequence in cell culture has been found to lead to increased protein expression and/or titer (g/L) of the POI and/or productivity in a specific (pg/cell/d) as well as volumetric (g/L/h) manner (cf. the Examples below).

In another preferred embodiment, in structure (II) as defined above, Sequence C is SEQ ID NO: 38 and Sequence D is SEQ ID NO: 32 or *vice versa.* In this embodiment, it is preferred that SEQ ID NO: 9 is used. In the former configuration (Sequence C is SEQ ID NO: 38 and Sequence D is SEQ ID NO: 32), structure (II) as a whole has the nucleotide sequence of SEQ ID NO: 39, preferably the nucleotide sequence of SEQ ID NO: 40. Thus, in a preferred embodiment, structure (II) as defined above is SEQ ID NO: 39, preferably SEQ ID NO: 40. Respective constructs are designated herein as cSbgA. In this embodiment, the nucleic acid of the present invention comprises specific sequences that target the BGH polyA signal sequence (SEQ ID NOs: 38 and 32). Expression of this coding sequence in cell culture has been found to lead to increased protein expression and/or titer (g/L) of the POI and/or productivity in a specific (pg/cell/d) as well as volumetric (g/L/h) manner (cf. the Examples below).

In another preferred embodiment, in structure (II) as defined above, Sequence C is SEQ ID NO: 41 and Sequence D is SEQ ID NO: 42 or *vice versa.* In this embodiment, it is preferred that SEQ ID NO: 51 is used. In the former configuration (Sequence C is SEQ ID NO: 41 and Sequence D is SEQ ID NO: 42), structure (II) as a whole has the nucleotide sequence of SEQ ID NO: 43, preferably the nucleotide sequence of SEQ ID NO: 44. Thus, in a preferred embodiment, structure (II) as defined above is SEQ ID NO: 43, preferably SEQ ID NO: 44. Respective constructs are designated herein as cScmP. In this embodiment, the nucleic acid of the present invention comprises specific sequences that target the hlgG (human IgG) light and heavy chain 5'-UTR (SEQ ID NOs: 41 and 42). Expression of this coding sequence in cell culture has been found to lead to increased protein expression and/or titer (g/L) of the POI and/or productivity in a specific (pg/cell/d) as well as volumetric (g/L/h) manner (cf. the Examples below).

In another preferred embodiment, in structure (II) as defined above, Sequence C is SEQ ID NO: 45 and Sequence D is SEQ ID NO: 46 or *vice versa.* In this embodiment, it is preferred that SEQ ID NO: 9 is used. In the former configuration (Sequence C is SEQ ID NO: 45 and Sequence D is SEQ ID NO: 46), structure (II) as a whole has the nucleotide sequence of SEQ ID NO: 47, preferably the nucleotide sequence of SEQ ID NO: 48. Thus, in a preferred embodiment, structure (II) as defined above is SEQ ID NO: 47, preferably SEQ ID NO: 48. Respective constructs are designated herein as cShyA. In this embodiment, the nucleic acid of the present invention comprises specific sequences that target human CYP1A1 mRNA (SEQ ID NOs: 45 and 46). Expression of this coding sequence in cell culture has been found to lead to increased reporter protein expression (measured as relative light units (RLU)) of the reporter protein and/or productivity in a specific (pg/cell/d) manner (cf. the Examples below).

In particular embodiments, the nucleic acids of the present invention independently comprise at least two of the structures (I) and (II) as defined above. Respective structures are termed dual Snord species or dual Snords herein. In further embodiments, the nucleic acids of the present invention independently comprise at least 3, *i.e.,* 3, 4, 5, 6, 7, 8, 9, 10, 11, 12 or more of the structures (I) and (II) as defined above, wherein the presence of 3, 4, 6, 8, 9 or 12 of the structures (I) and (II) as defined above is preferred. In preferred embodiments, the structures (I) and (II) as defined above are separated by a nucleic acid capable of forming a stem-loop-stem structure. Respective stem-loop-stem structures and nucleic acids capable of forming the same are not particularly limited and are known in the art. In particular, sequences capable of forming a stem structure include sequences such as 5'-(CTT)₃, 3'-(AAG)₃, 5'-(CAA)₃, and 3'-(TTG)₃. These can be combined with sequences capable of forming a loop structure as known in the art. In particular embodiments, the sequences flanking the structures (I) and (II) as defined above, i.e., the sequences according to SEQ ID NO: 1, SEQ ID NO: 5, SEQ ID NO: 7, and the sequence CTGRAGAA in the dual Snords according to the present invention, can be partly replaced by respective sequences capable of forming a stem structure and/or respective sequences capable of forming a loop structure.

In a particular embodiment, the present invention relates to a dual Snord combining the above constructs cSgIA and cS74, said dual Snord being designated as dicSgl74. This dual construct preferably comprises or consists of the nucleotide sequence of SEQ ID NO: 49, more preferably the nucleotide sequence of SEQ ID NO: 50. In a further embodiment, the present invention relates to a tetrameric Snord based on dicSgl74 and further targeting the 3'-UTR of a luciferase reporter gene, human HSP90AA1 mRNA and human SSRP1 mRNA, said tetrameric Snord being designated as XcSgl74h9ss. This tetrameric construct preferably comprises or consists of the nucleotide sequence of SEQ ID NO: 54, more preferably the nucleotide sequence of SEQ ID NO: 55.

The present invention expressly includes isolated nucleic acids having at least 65% sequence identity to the isolated nucleic acids defined above. More preferable are sequence identities of at least 67.5%, at least 70%, at least 72.5%, at least 75%, at least 77.5%, at least 80%, at least 82%, at least 84%, at least 86%, at least 88%, at least 90%, at least 91 %, at least 92%, at least 93%, at least 94%, at least 95%, at least 95.5%, at least 96%, at least 96.5%, at least 97%, at least 97.5%, at least 98%, at least 98.2%, at least 98.4%, at least 98.6%, at least 98.8% at least 99%, at least 99.1 %, at least 99.2%, at least 99.3%, at least 99.4%, at least 99.5%, at least 99.6%, at least 99.7%, at least 99.8%, or at least 99.9%, wherein higher identities are more preferred. This applies to the nucleic acids according to structures (I) and (II) as defined above as a whole, as well as to the individual sequence elements thereof, *i.e.,* to SEQ ID NOs: 1 to 35, and 38 to 58, as well as to the sequence CTGRAGAA present in structure (II) as defined above, and Sequences A to D. In this context, each individual sequence element may have a different sequence identity to the sequence elements defined above. By way of example, a nucleic acid according to the present invention based on structure (I) as defined above may comprise a sequence having e.g. 80% sequence identity to SEQ ID NO: 1, followed in 5'- to 3'-direction by a sequence having e.g. 98% sequence identity to SEQ ID NO: 12 (Sequence A), followed by a sequence having e.g. 75% sequence identity to SEQ ID NO: 3, followed by a sequence having e.g. 82% sequence identity to SEQ ID NO: 13 (Sequence B), followed by a sequence having e.g. 99% sequence identity to SEQ ID NO: 5.

In preferred embodiments, the isolated nucleic acids of the present invention are recombinant nucleic acids, *i.e.,* nucleic acids that are formed by laboratory methods of genetic recombination, such as e.g. molecular cloning.

The nucleic acids of the present invention can be transiently expressed in a host cell, or can be stably integrated into a host cell genome. In this context, the nucleic acids of the present invention can be flanked in 5'- and/or 3'-positions by functional sequences such as e.g. restriction sites, intronic sequences, regulatory sequences (3'- and/or 5'-UTR regions) and/or coding sequences, and transcription attenuation sequences (e.g. polyadenylation sites). In preferred embodiments, the nucleic acids of the present invention are present as intronic sequence within the coding sequence of a particular gene, e.g. a gene of interest. Accordingly, said nucleic acids can be flanked by intron sequences, e.g. minimal intron sequences, which are not particularly limited and are known in the art. An example of a 5'-intron sequence is the sequence GTAAGTR. Further, an example of a 3'-intron sequence is the sequence according to SEQ ID NO: 36, preferably the sequence according to SEQ ID NO: 37. In this context, according to a preferred embodiment, the nucleic acids of the present invention are present as an intronic sequence within the coding sequence of a gene encoding a factor that provides resistance against an antibiotic such as puromycin, hygromycin b, Zeocin^{®}, geneticin G418, blasticidin, phleomycin, DHFR and/or Glutamine synthetase. In this manner, the presence and translation of a nucleic acid of the present invention in a host cell can be easily controlled for by cultivating the host cell in the presence of the respective antibiotic. In general, the nucleic acids of the present invention are applicable as an intronic sequence in any coding sequence, non-coding sequence and/or untranslated region (5'- and/or 3'-UTR).

In a second aspect, the present invention relates to a vector comprising at least one nucleic acid according to the present inventions. In this context, the term "vector" relates to any vehicle that can artificially carry genetic material into a cell, where it can be replicated and/or expressed. Respective vectors are not particularly limited and are known in the art. They preferably include plasmid vectors, viral vectors, and bacterial vectors, wherein plasmid vectors, in particular plasmid expression vectors, are preferred. Suitable plasmid expression vectors that can be used in the context of the present invention are not particularly limited and are known in the art.

In a preferred embodiment, the nucleic acids of the present invention are present in the vectors of the present invention as an intronic sequence within the coding region of a gene of interest as defined above.

In a third aspect, the present invention relates to a host cell comprising at least one nucleic acid of the present invention and/or at least one vector of the present invention.

Suitable host cells are not particularly limited and include prokaryotic and eukaryotic organisms, cells, and cell lines. Prokaryotic cells are preferably selected from bacterial and archaeal cells. Eukaryotic cells are preferably selected from the group consisting of plant cells, insect cells, fungal cells, including yeast cells, and mammalian cells. Preferably, the host cells of the present invention are derived from mammalian cell lines, preferably selected from the group consisting of CHO cells, BHK-21 cells, PerC6 cells, CAP cells, HEK293 cells, HeLa cells, NS0 cells, SP2/0 cells, YB2/0 cells, EB66 cells, hybridoma cells, Jurkat cells, H4IIE cells, HepG2 cells, Hepa1 c1 c7 cells, HepaRG cells, U2OS cells, T47D cells, MCF-7 cells, H295R cells, LNCaP cells, amniocytes, and omnipotent, pluripotent, multipotent as well as oligopotent mammalian stem cells, wherein CHO cells, in particular CHO-K1 cells, CHO-DG44 cells, CHO-S cells, and CHO-DP12 cells are especially preferred. In other preferred embodiments, the host cells of the present invention are derived from aquatic vertebrate cell lines, preferably selected from the group consisting of RTL-W1 cells, ZFL cells, and RTG-2 cells. In further preferred embodiments, the host cells of the present invention are derived from other eukaryotic cell lines, preferably selected from the yeast group consisting of *Saccharomyces cerevisiae, Pichia pastoris,* and *Candida albicans.* In yet further preferred embodiments, the host cells of the present invention are derived from other eukaryotic cell lines, preferably selected from the algae group consisting of *Chlamydomonas reinhardtii, Scenedesmus obliquus,* and *Phaeodactylum tricornutum.* In yet further preferred embodiments, the host cells of the present invention are derived from other eukaryotic organisms and/or cell lines derived from higher plants, e.g. selected from the group consisting of *Arabidopsis thaliana, Nicotiana tabacum, Nicotiana rustica,* other *Nicotiana spp., Linum usitatissimum, Gossypium spp., Cannabis sativa, Zea mays, Oryza sativa, Glycine max, Lens culinaris, Triticum spp.* and *Solanum tuberosum.* In yet further preferred embodiments, the host cells of the present invention are derived from other eukaryotic cell lines derived from filamentous fungi, e.g. selected from the group consisting of *Aspergillus spp., Rhizopus spp., Trichoderma spp.* or *Penicillium spp..* In yet further preferred embodiments, the host cells of the present invention are derived from other eukaryotic cell lines derived from insects, e.g. selected from the group consisting of Sf9 cells and Sf21 cells derived from *Spodoptera frugiperda,* cell lines derived from *Bombyx mori, Mamestra brassicae* and *Drosophila melanogaster,* and BTI-Tn-5B1-4 cells derived from *Trichoplusia ni.* In yet further preferred embodiments, the host cells of the present invention are derived from prokaryotic cells, preferably selected from archaea such as *Sulfolobus spp., Natronococcus spp., Halobacterium spp., Pyrococcus spp.* or *Haloarcula spp..*

The nucleic acids of the present invention can be transiently expressed in the host cells of the present invention, or can be stably integrated in the host cells genome. In this context, said nucleic acids are preferably present as an intronic sequence within the coding region of a gene of interest as defined above.

The host cells of the present invention are preferably cells that can be used as a production cell line for the expression of a protein of interest, or as reporter cell line, e.g. in toxicology and target screening, or for increased production efficiency of products such as lipids, hydrogen, enzymes and/or biomass e.g. derived from cell lines, yeast, fungi, algae or plants.

In a fourth aspect, the present invention relates to a method for protecting and/or stabilizing an RNA of interest in a host cell, comprising the step of expressing at least one nucleic acid according to the present invention in said host cell.

In this aspect, the nucleic acid comprises specific sequences (Sequences A and B, or Sequences C and D) that are specific for the RNA of interest, *i.e.,* that hybridize to the RNA of interest and mediate the target stabilization. The nucleic acid can be part of a vector of the present invention. Further, the host cell can be a host cell of the present invention.

The RNA of interest is preferably selected from the group of mRNA, ncRNAs, snRNA, tRNA, miRNA and rRNA. In a preferred embodiment, the nucleic acid is present as an intronic sequence within the coding region of the RNA of interest.

In a particular embodiment, the RNA of interest is an mRNA, and protecting and/or stabilizing said mRNA in the above method of the present invention results in increased expression of the protein encoded by said mRNA.

Means for expressing a nucleic acid of the present invention in a host cell are nor particularly limited and are known in the art.

In this aspect of the present invention, all relevant definitions, limitations and embodiments defined herein apply in an analogous manner.

In a fifth aspect, the present invention relates to a related method for expression of a protein of interest (POI), comprising the step of coexpressing at least one nucleic acid of the present invention with the coding sequence of said POI in a host cell.

In this aspect, the nucleic acid comprises specific sequences (Sequences A and B, or Sequences C and D) that are specific for the mRNA encoding said POI, *i.e.,* that hybridize to said mRNA. The nucleic acid can be part of a vector of the present invention. Further, the host cell can be a host cell of the present invention.

In a preferred embodiment, the nucleic acid is present as an intronic sequence within the coding region of the above POI.

Means for coexpressing a nucleic acid of the present invention with a coding sequence of a POI in a host cell are nor particularly limited and are known in the art.

In this aspect of the present invention, all relevant definitions, limitations and embodiments defined herein apply in an analogous manner.

In a sixth aspect, the present invention relates to a method of increasing the cell density (cells/ml) and/or the specific productivity (pg/cell/day) and/or the volumetric productivity (g/L/h) and/or the titer (g/L) and/or the reporter or enzyme activity (measured as relative fluorescence units (RFU), relative light units (RLU), or absorbance) of a protein of interest (POI) in a culture of host cells, comprising the step of expressing at least one nucleic acid according to the present invention in said host cells.

The nucleic acid can be part of a vector of the present invention. Further, the host cell can be a host cell of the present invention.

In this aspect of the present invention, all relevant definitions, limitations and embodiments defined herein apply in an analogous manner.

In final aspects, the present invention relates to uses of the nucleic acids of the present invention for stabilizing an RNA of interest in a host cell, expressing a protein of interest (POI) in a host cell, or increasing the cell density and/or cell viability in a culture of host cells.

In these aspects, all relevant definitions, limitations and embodiments defined herein, in particular with respect to the methods of the present invention, apply in an analogous manner.

In preferred embodiments, the methods and uses of the present invention are performed *in vitro, i.e.* they are not performed on the human or animal body. However, said methods and uses are capable of being performed *in vivo,* preferably in plants, and can further be used for medical purposes. Thus, the present invention relates to the nucleic acids of the present invention for use in medicine.

The term "overexpression" as used herein with respect to the expression product of a host cell, such as a recombinant protein of interest (POI), shall mean the increase of the expression product as obtained in a host cell culture. For example, a soluble expression product may be determined to be overexpressed by the increased amount in the culture medium, or a membrane-bound product may be determined to be overexpressed by the increased amount in a cell fraction. The term specifically applies to the amount that is at least 2-fold, preferably at least 5-fold, preferably at least 25-fold, more preferably at least 100-fold, as compared to a wild type host cell, e.g. expressing at normal levels.

Such overexpression of at least one isolated nucleic acid of the invention in a host cell may result in the increase of the yield of a recombinant POI, reporter protein or biomass produced by the same host cell. The host cell as used according to the invention specifically is recombinantly engineered to overexpress both, the isolated nucleic acid of the invention and the POI, herein also referred to as co-overexpression. More preferentially, the isolated nucleic acid of the invention intronically locates in the POI.

To overexpress any of the isolated nucleic acids of the invention and/or the POI inducing the isolated nucleic acids of the invention may be through addition or co-expression of expression enhancers or helper factors. Expression enhancers are e.g. enhancer as well as intronic sequences upstream and downstream of promoter sequences respectively (e.g. chEF-1 promoter, hEF1a promoter, SV40 promoter, PGK promoter, CMV, CMVᵢₑ (immediate-early enhancer/promoter)) as well as intronic sequences, an IRES (internal ribosomal entry site) and strong polyadenylation sequences (e.g. BGH, SV40) for proper transcription termination. Alternative measures to overexpress any of the isolated nucleic acids of the invention and/or the POI inducing the isolated nucleic acids of the invention may be through inducible reporter systems such as hormonally (e.g. estrogen-, androgen- and/or glucocorticoid-response elements), else chemically (e.g. Tet on/off, Methallothionin/Zn²⁺) and/or intrinsic protein- and/or RNA-activated expression cassettes.

A preferred expression system employs a vector comprising a CMVᵢₑ (immediate-early enhancer/promoter), a resistance gene (e.g. against Zeocin^{®} or geneticin G418 or hygromycin b or puromycin or phleomycin or DHFR or Glutamine synthetase) inducing the isolated nucleic acids of the invention and a final polyadenylation sequence (e.g. BGH, SV40, synthetic) for proper transcription termination.

The term "comprise(s)/comprising" as used herein is expressly intended to encompass the terms "consist(s)/consisting essentially of" and "consist(s)/consisting of".

The present invention is closing a gap in biotechnology. In particular, specific and targeted means for the stabilization of RNA species have so far not been available. With the present invention, the expression of protein products and/or the activity of any target RNA species of interest can be increased in a highly specific and targeted manner. Specific 2'-O-methylation of target RNAs with the help of the snoRNA-derived nucleic acids of the present invention leads to increased retention time and/or increased activity of said RNAs. This can be employed for modulating mRNA stability and/or translation capacities via improved maturation of ribosomal RNA and/or the spliceosome.

The present invention is simple and efficient in use. Since snoRNAs are found in all eukaryotes and also in some archaea, the present invention can be used for a broad range of applications. Possible applications can be conceived in white, red and green biotechnology, in order to realize novel and improved products. These include e.g. increasing the expression of therapeutic or biopharmaceutical proteins, the modulation of cells for synthetic biotechnology, increasing the performance of reporter cell lines used in toxicology, as well as a number of applications in basic research. Further, a wide range of therapeutic applications seems to be possible.

The nucleic acids of the present invention can be incorporated as intronic sequences into any particular gene of interest. Further, different nucleic acids according to the present invention can be combined in expression cassettes. Thus, the present invention provides economic and efficient means for increasing the expression of genes and/or stabilizing and/or protecting other regulatory or process-relevant RNA species in a universal manner.

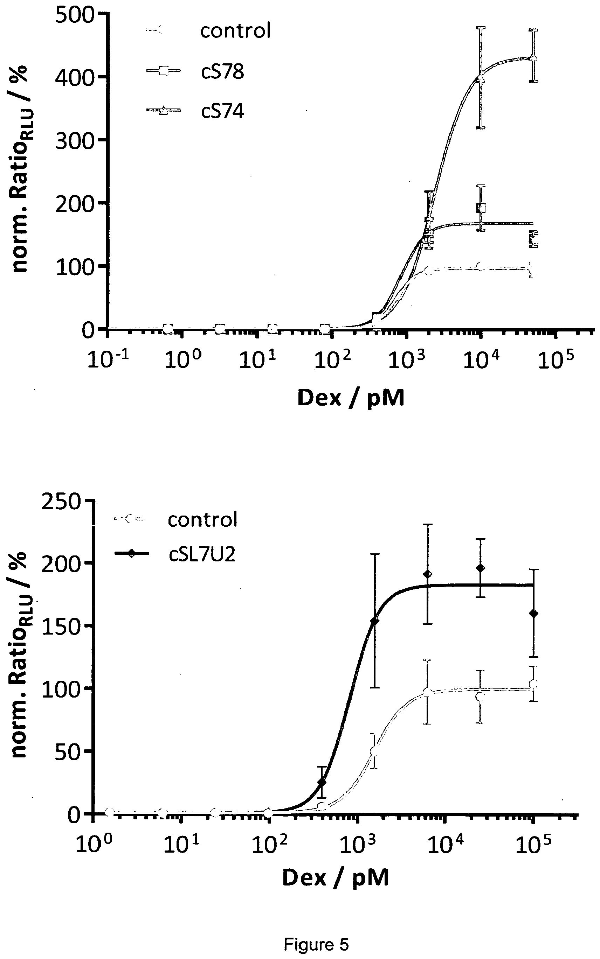

The figures show:
Figure 1:
   Overview of the coding region for the *Cricetulus griseus* ncRNA Gas5. Exons are shown in dark gray, introns in light red, and Snords in light gray. The sequences of Snord74 (cS74) and Snord78 (cS78) are shown.
Figure 2:
   Structure of Snords cS74 and cS78. The Snords were cloned between a codon-optimized puromycin resistance gene (puromycin N-acetyl transferase; shaded in black) and are flanked by Spel- and *Age*l-cleavage sites (orange letters shaded in light gray). Upstream and downstream of these cleavage sites, minimal intron sequences are present (dotted; including the polyC/T region). The snoRNA-specific C- and D-boxes are shaded in orange. The specific sequences for both species are shaded in gray (cS78) and red (cS74). These specific sequences are responsible for RNA recognition.
Figure 3:
   Variations of the native cS78 sequence. The cS78 specific sequences (shaded in green) were optimized for CHO 28S and 18S rRNA (cS2818), or for CHO Luc7 mRNA and spliceosomal U2 snRNA (cSL7U2).
Figure 4:
   Overexpression of Snords cS78, cS2818, cSL7U2, and cS74 in CHO-DP12 cells. cS2818 increases hlgG productivity versus mock control (left panel). cSL7U2 and cS2818 show a lower productivity compared to cS78, with cS74 showing the highest productivity (right panel). cSL7U2 showed a 44% increased cell viability compared to cS78 (data not shown).
Figure 5:
   Application in reporter cell lines. cS74 and cSL7U2 were stably transcribed in a glucocorticoid-sensitive reporter cell line (GR-CALUX). Both species induced an increase in induction strength via increased generation of *Photinus pyralis* luciferase. In addition, cSL7U2 showed a decreased EC₅₀ value and, thus, an increase in sensitivity.
Figure 6:
   Luciferase-specific Snord cSgIA. cSgIA is based on cS78 and has been adapted to the 3'-UTR (untranslated region) of the luciferase gene of reporter cell line GR-CALUX. The wildtype Snord and a Snord that is not specific for the reported cell line were used as controls.
Figure 7:
   Luciferase-specific Snord cSgIA in combination with cS74 or used on two cell lines, respectively. cS74 increased the specific effect of cSgIA in the cell lines GR-CALUX (Dex; upper panel) and ERα-CALUX (E2; lower panel).
Figure 8:
   Verification of specific mRNA stabilization by cSL7U2 and a Gluc-coupled tandem-antisense-anchor. A synthetic, codon-optimized gene for *Gaussia* luciferase (Gluc) was furnished with a tandem-antisense-linker (a-Linker) that is complementary to cSL7U2 (SLU). The linker was inserted after the Gluc coding sequence and before the polyadenylation signal und comprised only the Luc7-specific region of cSL7U2. Expression of Gluc was controlled by estrogen response elements (ERE) in four repetitions. To verify specific stabilization of the Gluc mRNA, four constructs were provided, wherein three of those constructs served as controls. EGP: control without *a*-Linker, without SLU (standard control); EGaP: control with *a*-Linker, without SLU (control for unspecific *a*-Linker binding); EGSLU: control without *a*-Linker, with SLU (control for the visualization of SLU-specific Gluc activation); EGaSLU: specific construct with *a*-Linker and SLU. The U2 snRNA-specific sequence remained untouched.
Figure 9:
   Verification of specific mRNA stabilization by cSL7U2 and a Gluc-coupled tandem-antisense-anchor. Transfection with linearized constructs (lin) was stably performed in in the cell line ERα-CALUX (E2) with intrinsic luciferase activity upon activation of the ERα receptor. EGaSLU showed increased expression of Gluc (A) and CALUX (B) activity under influence of estradiol (E2). In contrast, EGSLU increased only CALUX activity, to a lesser extent. Both controls without cSL7U2 showed identical behavior. This is consistent with a specific effect of cSL7U2 on the *a*-Linker, wherein the former element increases intrinsic luciferase activity at the same time (analogous to the data shown in Fig. 5).
Figure 10:
   (A) cSbhA which is specific for the BGH (bovine growth hormone) polyA signal, was designed in an analogous manner to cSgIA. In a CHO-K1-based cell clone (CHO1H7), producing a monoclonal antibody (mAb), a slight increase of mAb production in a fed-batch process could be shown. CHO1H7 showed the highest production activity, indicating a high RNA stability. Data points are based on four biological and three technical replicates each. (B) Expression of cSbhA in a precursor cell pool of the production clone CHO1H7. A higher increase of mAb production was seen in a fed-batch process compared to the high-producing clone CHO1H7. Thus, cSbhA can increase the fraction of high-producing subpopulations during especially in the early phase of cell line development. Data points represent three biological and three technical replicates each.
Figure 11:
   Switch of intrabox sequences and application in production cell clone CHO1H7. The hlgG titer is increased versus the control in both cSbhA and its analog cSbgA, wherein the titer for cSbgA is even slightly higher than the titer for cSbhA. Cell density at day 0 was 7×10⁶/ml, incubation for 39 h. Data points represent three biological and four technical replicates each.
Figure 12:
   (A) Structure of Snord cScmP. (B) Application at the 5' UTR of hlgG transcripts in production cell clone CHO1H7. The hlgG titer is not increased versus the control using cScmP. However, due to the reduced cell density during the complete fed-batch process, an increased specific productivity is achieved. Data points represent three biological and three technical replicates each. (C) Application at the 5' UTR of hlgG transcripts in production cell clone CHO1H7. At a high initial cell density, the hlgG titer is increased by cScmP versus the control. Cell density at day 0 was 6×10⁶ml, incubation for 24 h. Data points represent three biological and four technical replicates each.
Figure 13:
   (A) Pathway engineering. Structure specific recognition protein 1 (Ssrp1) was detected based on highly producing clone, wherein this gene was constitutively activated by insertion of the foreign gene. cSssA, a construct for Ssrp1 with high homology for diverse species was designed based on cS74. (B) cSssA increased the productivity and viability of CHO1H7 in a fed-batch process.
Figure 14:
   (A) CYP1A1-specific Snord cShyA for stabilizing human CYP1A1 mRNA. (B) CYP1A1-specific Snord cShyA and increase of metabolic capacity. The construct cShyA effects a higher CYP1A1 activity in the estrogen response reporter cell line T74Dkbluc (ATCC® CRL-2865™), whereby the agonist Benzo[a]pyrene (B[a]P) is metabolized to a higher extent. The hydroxylated form of B[a]P shows estrogenic activity, inducing the transgenic *P. pyralis* luciferase (Pluc). Therefore, the increased CYP1A1 activity leads to an increased Pluc induction.
Figure 15:
   (A) Combination of Snords cSgIA and cS74 to dicSgl74. For the design of dicSgl74, crystal structures of the Nop5/fibrillarin-complex were used (DOI: 10.1093/nar/gkw576). The stem structures of cSgIA (cS78-based, respectively) and cS74 where replaced by flanking 5'-(CTT)₃ and 3'-(AAG)₃, and 5'-(CAA)₃ and 3'-(TTG)₃, respectively, which form stable stem structures. The flexible loop between the both stem structures hypothetically affords the ideal fit into the Nop5//fibrillarin-complex. (B) Dual SNORD species dicSgl74 is more stable than the separated transcription of both consisting SNORDs using cSgl74. The luciferase induction by dexamethasone (Dex) is comparable between both species dicSgl74 and cSgl74. In contrast to dual SNORD dicSgl74, the transcription of each SNORD in a intronic sequence separately led to a lower luciferase stability at higher Dex concentration. Applying a nonlinear regression curve fit (dotted line), the extrapolation of diSgl74 is more representative and reliable. Each data point consist of six biological with each two technical replicates.

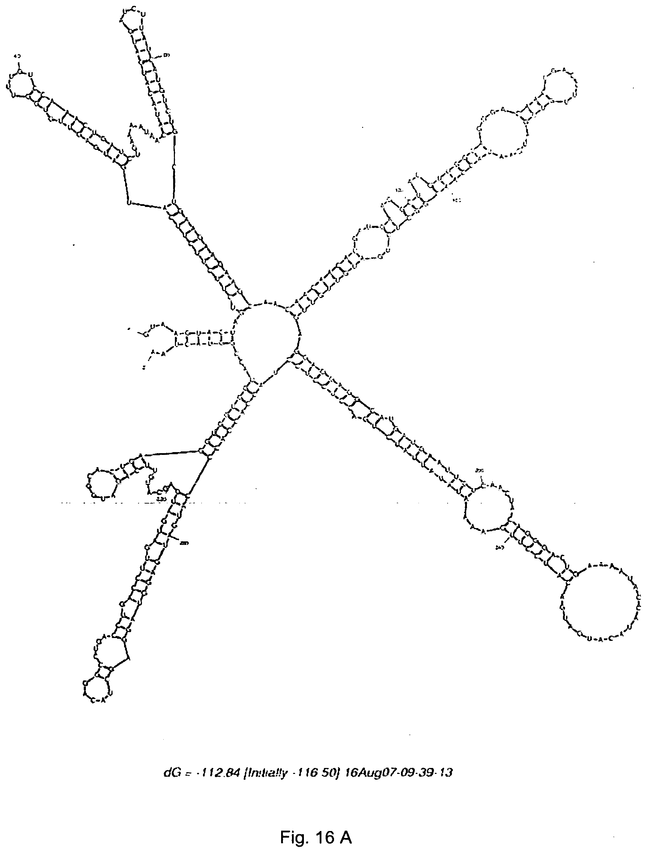

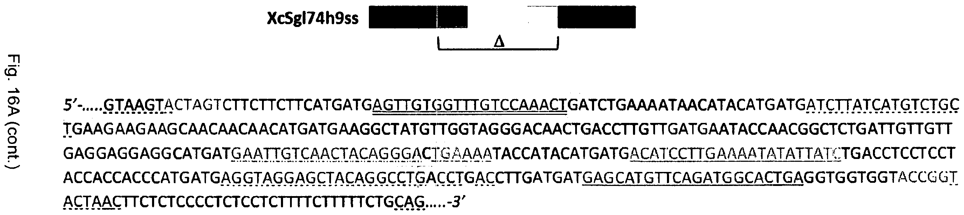

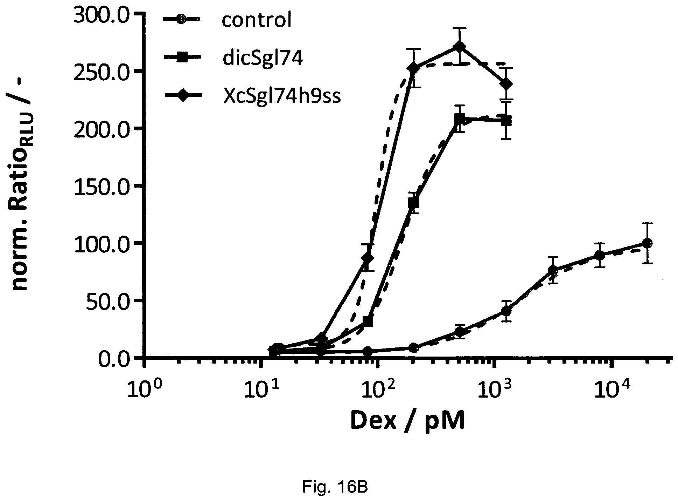

Figure 16: (A) Tetrameric SNORD XcSgl74h9ss targeting 3'UTR of luciferase reporter gene, human HSP90AA1 mRNA (NM_001017963.2) and human SSRP1 mRNA (NM_003146.2) as well as including cS74. Based on dicSgl74, a synthetic SNORD construct was developed to further improve nuclear receptor mediated reporter gene activation. Thereby, HSP90 was targeted to improve the ligand binding to the respective nuclear receptor (GR, ERα, ERβ, AhR, etc.) as well as overall protein folding. The human SSRP1 facilitates polymerase II mediated transcription (10.1073/pnas.1222198110). For stabilizing the SNORD structure, stem structures were introduced: (CTT)₃-Sgl-(AAG)₃, (CAA)₃-S74-(TTG)₃, (AGG)₃-Sh9-(CCT)₃ and (ACC)₃-Sss-(GGT)₃. (B) The tetrameric SNORD XcSgl74h9ss leads to better analytical efficiency compared to dual-SNORD dicSgl74. The luciferase induction by dexamethasone (Dex) in GR-CALUX is further improved applying the tetrameric SNORD XcSgl74h9ss compared to dual-SNORD dicSgl74. A nonlinear regression curve fit (dotted line) is applied and each data point consist of six biological with each two technical replicates

The present invention will be further illustrated by the following examples without being limited thereto.

### Examples

### Example 1:

Overexpression of native and the cS class of nucleic acids in the hlgG production cell lines CHO-DP12 and CHO1 H7 as well as in mammalian reporter cell systems.

### a) General methods

The nucleic acids of the invention can be synthesized *de novo* or amplified by PCR with the appropriate restriction sites as well as regulatory sequences. Molecular cloning can be performed by restriction digest/ligation and/or homologous recombination *in situ* and/or bacterially driven recombination.

Gene delivery can be done through electroporation or nucleofection (AmaxaNucleofector, Lonza, Cologne), by lipofection (delivery by liposome/DNA vehicles) or by delivery of precipitated DNA/M^{2+/3+} complexes (M^{2+/3+}: e.g. Ca²⁺, Sr²⁺, Mg²⁺, Al³⁺, Cr³⁺). Other useful methods are sonoporation, microinjection, ballistic DNA-delivery or protoplast fusion.

After gene delivery the cells can be selected by a resistance gene (e.g. against geneticin G418, hygromycin b, puromycin, Zeocin^{®}) as well as e.g. DHFR or Glutamine synthetase system for approx. 10-21 days. These selection genes can be expressed by a separated promoter (e.g. SV40, CMV, CMVᵢₑ (immediate-early enhancer/promoter), hEF1 a, chEF-1 a or directly controlled by the expression of the gene of interest (GOI) with an IRES sequence upstream to the resistance gene.

### b) Adherent cell culture methods

Adherent cell lines were cultured in 25 cm², 75 cm² or 125 cm² cell culture flasks (TPP, Transadingen, Switzerland; Greiner, Frickenhausen, Germany) and/or 6-, 12-, 24- or 96-well plates (TPP, Transadingen, Switzerland; Greiner, Frickenhausen, Germany) using DMEM, RPMI1641, DMEM/F12, L15 and Or MEM basal media (100 µl - 20 ml; Gibco, Darmstadt, Germany) with the respective essential supplements FCS (1 - 20%; e.g. Biowest Europe, Nuaillé, France) or hPL (1 - 10 %; e.g. PL Bioscience, Aachen, Germany) or Panexin NTA (1 - 10 %; e.g. PAN-Biotech, Aidenbach, Germany) or ITS (1x; Heidelberg, Germany). Cell line dependent, the culture media were supplemented with e.g. MEM NEAA, Glutamax or L-Glutamin, bovine or recombinant human Insulin, recombinant human EGF, recombinant human Transferrin and/or HEPES (Gibco, Darmstadt, Germany; Sigma-Aldrich Chemie GmbH, Munich, Germany).

The standard cultivation settings are, unless otherwise mentioned: 4.5-5.0 % CO₂, 36.8 ± 0.5°C, >90% rel. humidity. For passaging, the cells were trypsinated. Cell counts can be estimated by Neubauer haemocytometer (Brand, Wertheim, Germany) with or without prior trypsination with Trypsin 0.5%/EDTA (Gibco, Darmstadt, Germany) and erythrosine B (Roth, Karlsruhe Germany) or Trypan Blue (Gibco, Darmstadt, Germany) staining or counted by instrumental cell counters (Coulter Counter from Beckman, Krefeld, Germany; CASY, Cedex or Cellavista from Innovatis, Roche, Mannheim, Germany).
hlgG concentrations were measured by ELISA standard procedures. Each sample technical replicates (n=2-4) were applied.

### c) Suspension cell culture methods

The Batch and FedBatch cultures can be performed in TubeSpin Bioreactor 50 (TPP, Transadingen, Switzerland) in duplicates to quadruplicates (n_{biological} = 2-4) under identical conditions.

All cultures as described in the Examples section were performed in CDM4PerMAb (HyClone, Thermo, Logan, Utah) supplemented with 5 mM Glutamax (Gibco, Darmstadt, Germany). During a FedBatch process a feed ratio up to 30-35% can be applied using daily alternating addition of CellBoost6 50 g/L (HyClone, Thermo, Logan, Utah) and Glucose 100 g/L.

The standard cultivation settings are, unless otherwise mentioned: 5-15 ml Medium, 220 rpm, Shaker amplitude (e.g. DOS-20M, LTF Labortechnik GmbH & Co. KG, Wasserburg, Germany) of 20 mm, 4.5-5.0 % CO₂, 36.8 ± 0.5°C, Airation (BFT50): A-E.

Cell counts can be estimated by Neubauer haemocytometer (Brand, Wertheim, Germany) with or without prior trypsination with Trypsin 0.5%/EDTA (Gibco, Darmstadt, Germany) and erythrosine B (Roth, Karlsruhe Germany) or Trypan Blue (Gibco, Darmstadt, Germany) staining or counted by instrumental cell counters (Coulter Counter from Beckman, Krefeld, Germany; CASY, Cedex or Cellavista from Innovatis, Roche, Mannheim, Germany).

hlgG concentrations were measured by ELISA standard procedures. Each biological sample (n=2-4) technical replicates (n=2-4) were applied.

Titers, volumetric productivities as well as specific productivities of two or more cell lines were compared using the last data sets at viabilities above 65-75% with a maximal difference of ±5% viability between compared data sets.

### d) Assaying

Assays for determining reporter gene or enzyme activities, the desired cell line was incubated with different concentrations of receptor agonists (e.g. estradiol, dexamethasone, β-naphtoflavone), antagonists and/or metabolic shift inducers such as sodium butyrate or sodium valerate and the respective media in 96-well or 384-well plates (TPP, Transadingen, Switzerland; Greiner, Frickenhausen, Germany). Reporter protein as well as enzyme activities were determined by specific substrate conversion leading to light or fluorescence and/or colour shift (e.g. D-luciferin, coelenterazine, 7-resorufin esters) or by direct/indirect ELISA measurement of cellular products.

### Example 2:

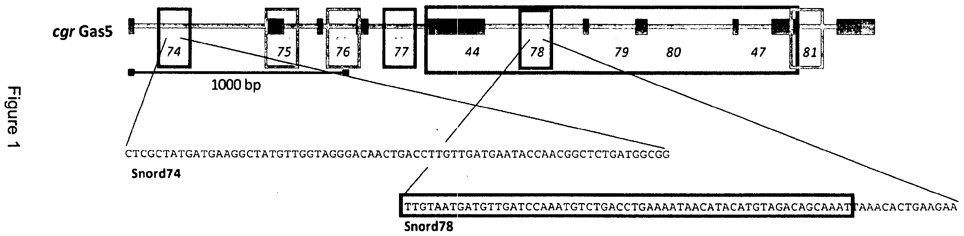

The invention is based on first observations of natural occurring C/D box small nucleolar RNA species (hereinafter called SNORDs). In prior, the *Cricetulus griseus* SNORDs Snord74, Snord75, Snord76, Snord77 and Snord81 (hereinafter called cS74, cS75, cS76, cS77 and cS81, respectively) derived from *Cricetulus griseus* Gas5 (cgrGas5) were stably transcribed in the antibody production clone CHO-DP12 to observe their potential effects on cell population behaviour (Figure 1).

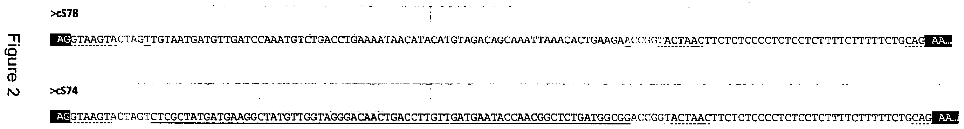

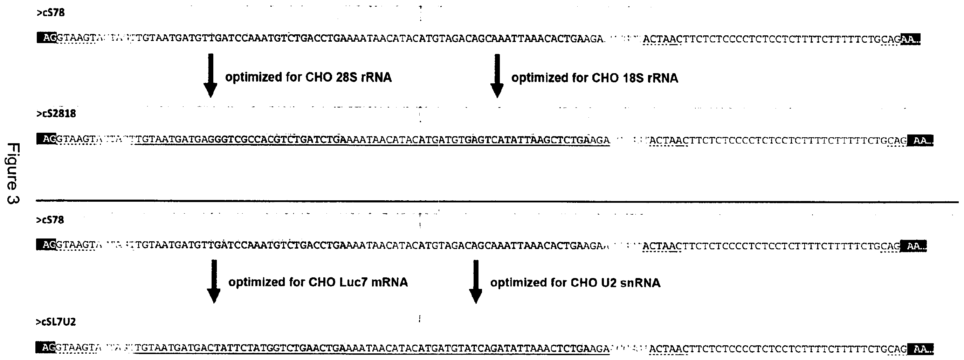

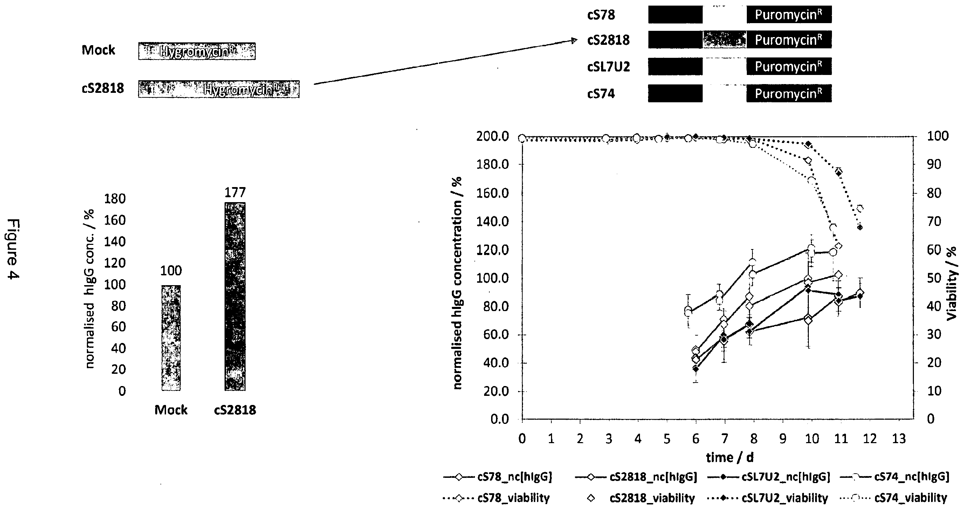

The constructs possessing cS74, cS77 and cS81 survived, but cS74 was able to generate proliferating subpopulation only. The cell size in cS77 transcribing cells was increased to a doubled cell's diameter to 14-21 µm (data not shown). All SNORDs from the cS series including the positive control cS78 were designed as minimal intronic sequences located within or prior to a resistant gene coding sequence (CDS) (Figure 2). Derived from cS78, the derivates cS2818 and cSL7U2 were designed according full homology to *Cricetulus griseus* sequences of ribosomal rRNA 28S (ref|NR_045212.1) and 18S (ref|NR_045132.1) or of the messenger RNA (mRNA) of Luc7 (ref|XM_003501549.1) and the small nuclear RNA (snRNA) U2.1 (ref|NW_003620837), respectively. Transcribed and overexpressed in CHO-DP12, cS2818 could increase the product titer (hIgG) 1.77-fold in direct comparison with the mock control (hygromycin resistance gene only) (Figure 4).

A separated independent approach using the puromycin resistance gene and the intronic sequences within the CDS was performed, whilst the synthetic constructs cS2818 and cSL7U2 resulted in a similar performance in productivity, which was slightly lower than the increase in productivity by cS78. Nevertheless, in both synthetic constructs and compared to cS78, the respective viable cell cultures were prolonged by one day (Figure 4) and integral viable cell densities were increased (cS2818: 1.18-fold; cSL7U2: 1.56-fold; ≥65 % viability). In contrast, the construct cS74 increased the product (hIgG) titer as well as the specific productivity by 1.22-fold and 1.41-fold, respectively. Applied on a glucocorticoid responsive reporter cell line (GR-CALUX; Figure 5), the fold change of *P. pyralis* luciferase activity was increased 1.57-fold (cS78), 1.92-fold (cSL7U2) and 4.54-fold (cS74), respectively. The introduction of the construct cSL7U2 let to a higher protein production (fold change induction) as well as a higher sensitivity (0.52-fold EC₅₀) compared to the positive control cS78.

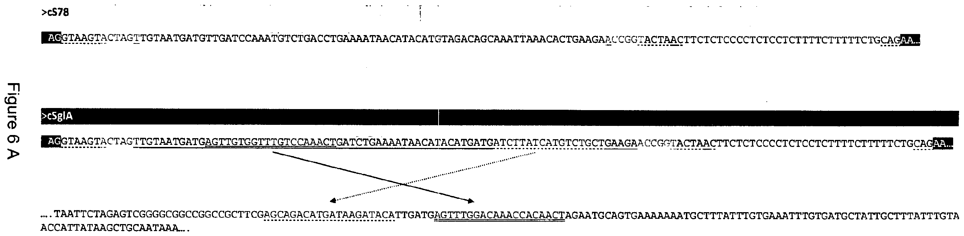

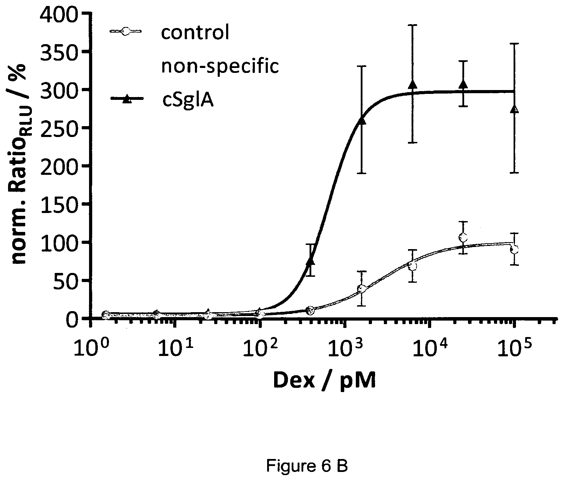

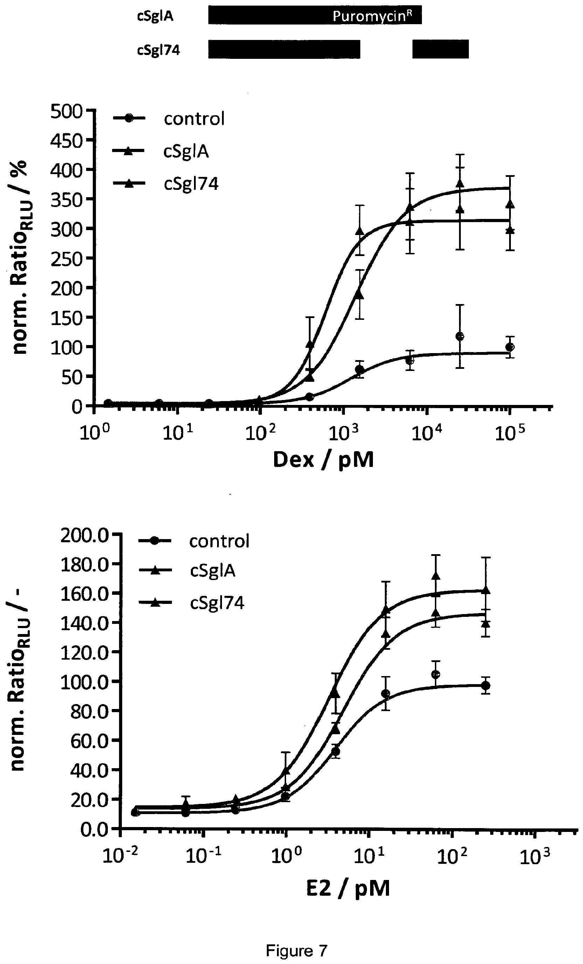

Subsequently, a *P. pyralis* Luciferase 3'-UTR specific synthetic SNORD-construct was designed and introduced into the same cell line (Figure 6A), which resulted in a 3.17-fold increase in luciferase induction and a 4.12-fold higher sensitivity (EC₅₀; Figure 6B). A scrambled, unspecific construct resulted in an insignificant change. Using a second intron possessing cS74 downstream the same puromycin resistance gene, the luciferase induction as well as the sensitivity could be further increased in the glucocorticoid-responsive (e.g. dexamethasone; Dex) reporter cell line (GR-CALUX) as well as the estrogen-responsive (e.g. estradiol; E2) reporter cell line (ERα-CALUX) (Figure 7).

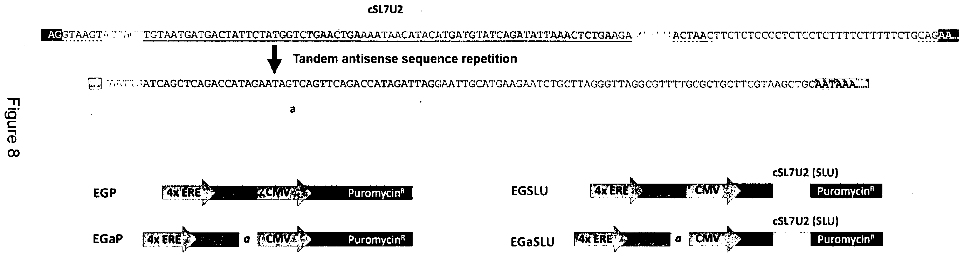

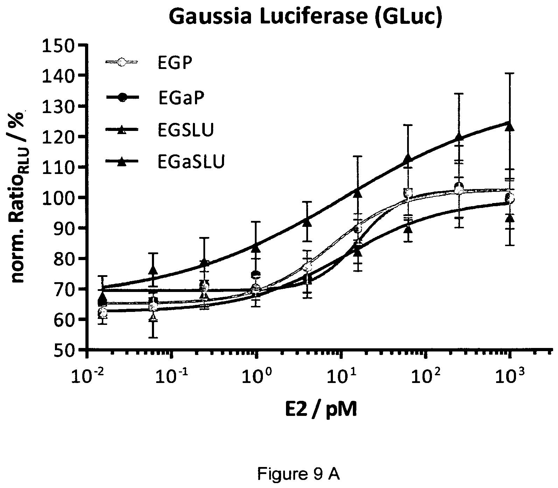

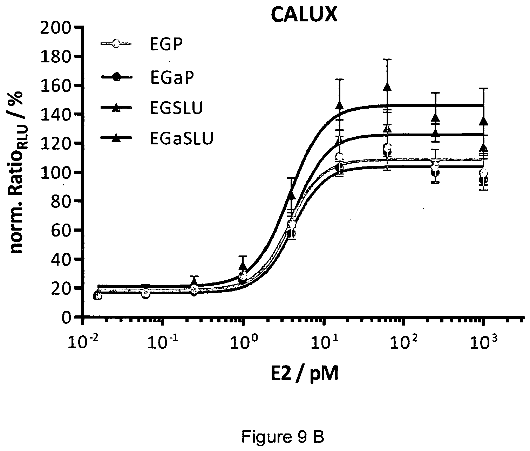

To further prove the specificity, a synthetic 3'-UTR of a *Gaussia* Luciferase controlled by four oestrogen-responsive enhancer elements was designed, which carried a doubled antisense sequence (a) for the Luc7-specific box of cSL7U2 (Figure 8). The resulting four constructs covering all possible variants (EGP: w/o a, w/o cSL7U2; EGaP: with a, w/o cSL7U2; EGSLU: w/o a, with cSL7U2; EGaSLU: with a, with cSL7U2) were introduced in the estrogen-responsive reporter cell line (ERα-CALUX). Here, both the internal *P. pyralis* and the introduced *Gaussia* luciferase activity was compared, revealing an unspecific increase in induction in the cell line possessing EGSLU as well as EGaSLU (P. *pyralis,* Figure 9B) and a specific increase in induction using EGaSLU only *(Gaussia,* Figure 9A).

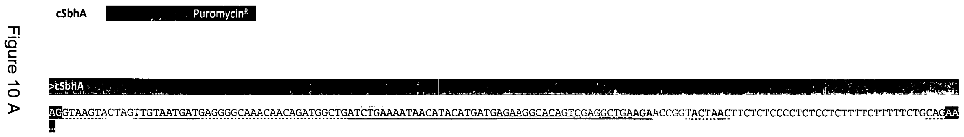

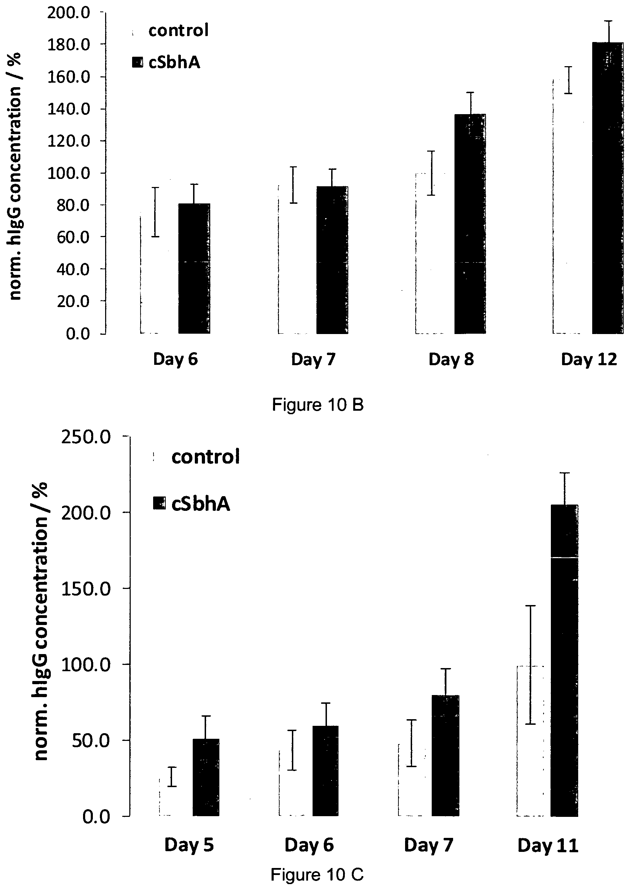

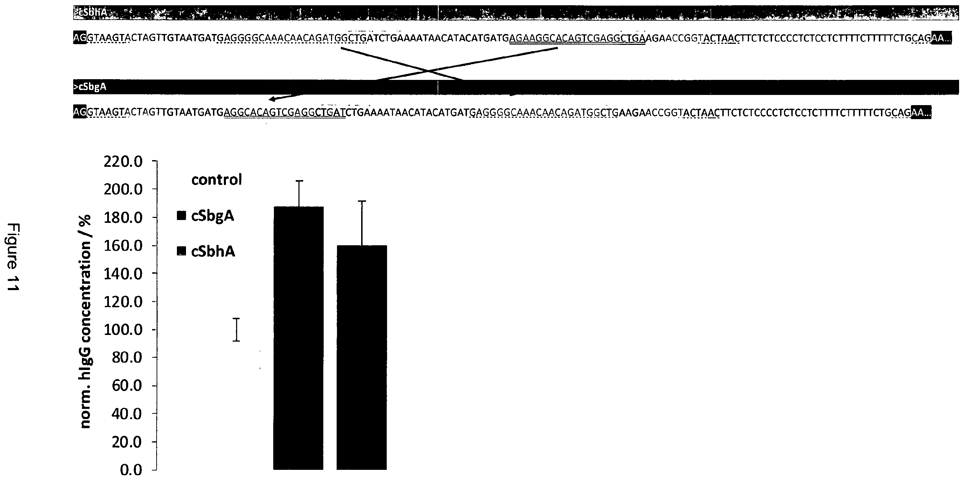

Using a CHO-K1 derived, hIgG-producing clonal suspension cell line (hereinafter called CHO1H7), the specific SNORD (cSbhA) binding the 3'-UTR for both CDS (coding sequences) of the encoding hlgG fragments was tested (Figure 10A). Hereby, the hIgG titer was increased (1.36-fold, day 8; 1.15-fold, day 12) applying a fed batch protocol (Figure 10B). Since the clonal cell line CHO1H7 could possess a high intrinsic transgene mRNA stability, the construct cSbhA was applied at its polyclonal progenitor cell line leading to a 2.06-fold increase in hlgG titer compared to the mock vector transfected control cell line (Figure 10C). Switching the target specific antisense boxes resulted in the construct cSbgA, which increased the hlgG product titer 1.88-fold (Figure 11). Here, the increase in productivity was slightly higher than applying cSbhA (1.17-fold; Ratio_{cSbhA/control}: 1.60-fold).

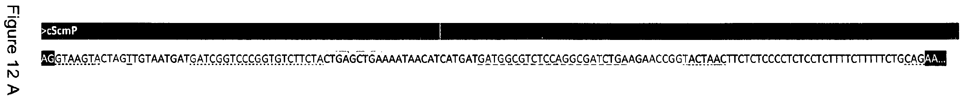

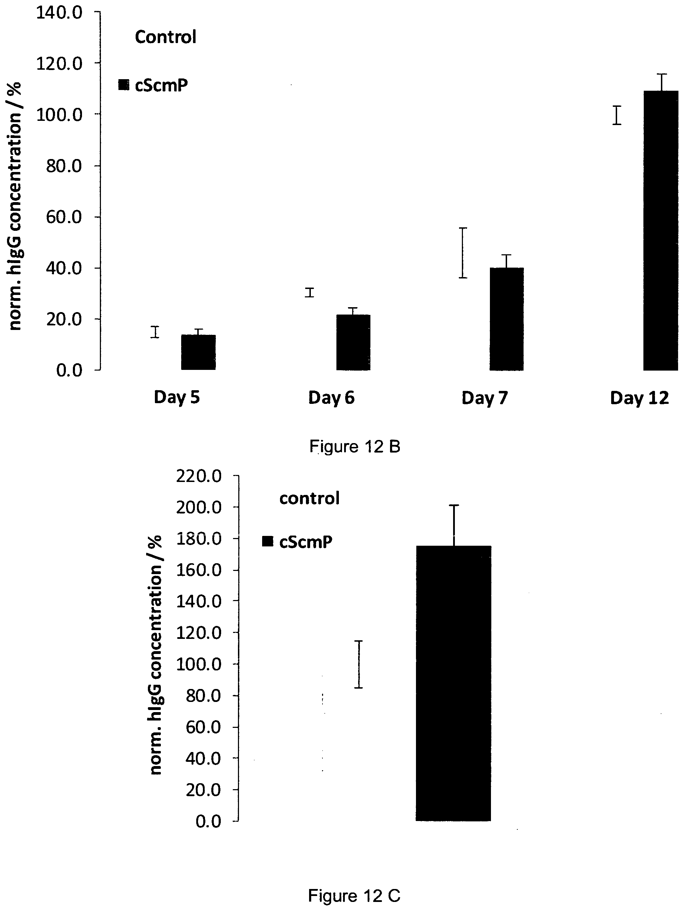

Applying the same strategy to the 5'-UTR of CHO1H7 hlgG transcripts, the construct cScmP is resulting (Figure 12A). During fed batch culture the hlgG titer was not increased compared to the control due to an overall reduced cell density (Figure 12B). In reverse, the cell specific productivity was increased (1.61-fold, ≥70 % viability). Because of this observation, an initially identical cell density was applied that resulted in a 1.76-fold increase in hlgG titer after 24 hours (Fig. 12 C).

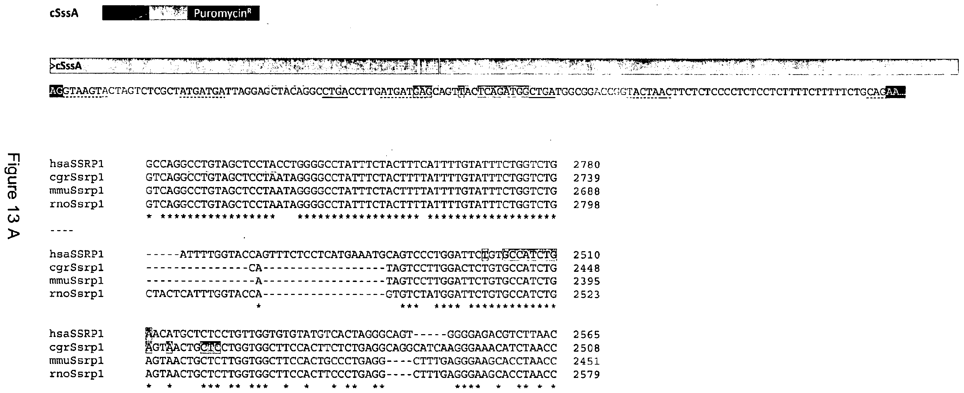

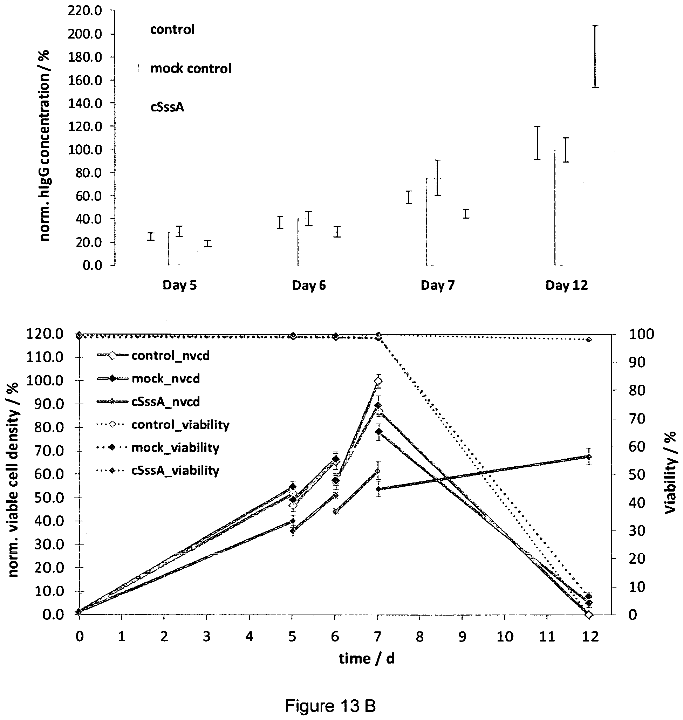

A SNORD construct (cSssA) for stabilising SSRP1/Ssrp1 (also FACT) was designed to overcome possible limitations in transcription/translation (Figure 13A). Once introduced in CHO1H7, the growth was decreased resulting a beneficial specific productivity (1.73-fold), whilst the product titer (2.38-fold, ≥90 % viability), integral viable cell density (1.84-fold, ≥90 % viability) and overall viability was increased (Figure 13B).

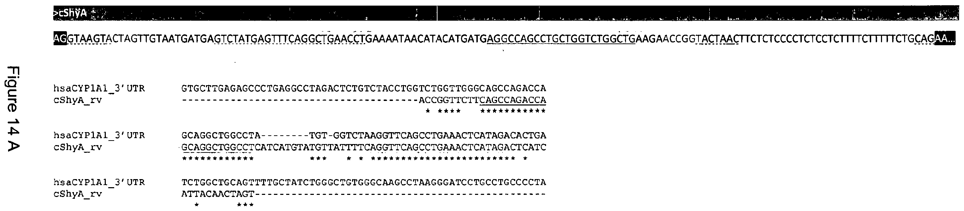

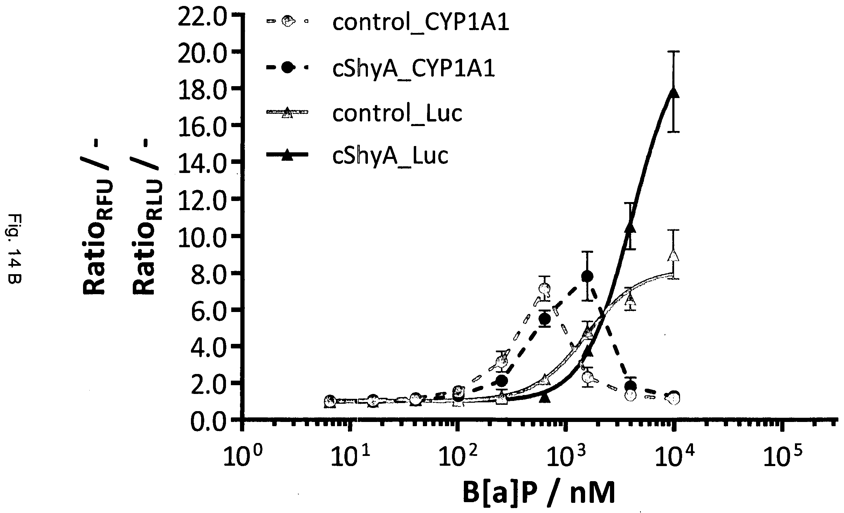

Using the estrogen-responsive reporter cell line T47Dkbluc, the CYP1A1-specific construct cShyA was verified (Figure 14A), whether an increased CYP1A1 activity, which results a higher metabolic capacity, leads to a higher luciferase induction due to an estrogenic activity of the applied benzo[a]pyrene (B[a]P). Indeed, a higher CY1A1 activity was observed even in higher B[a]P concentrations overcoming the competitive inhibition of CYP1A1 by B[a]P. Furthermore, the increased CYP1A1 activity led to a higher *P. pyralis* luciferase induction (2.55-fold).

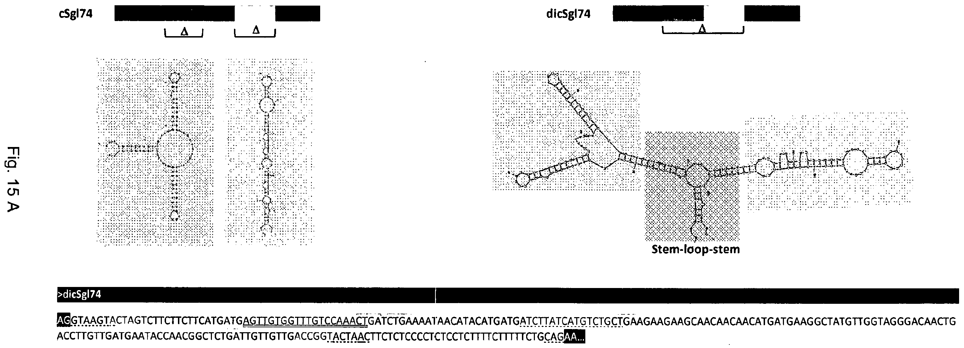

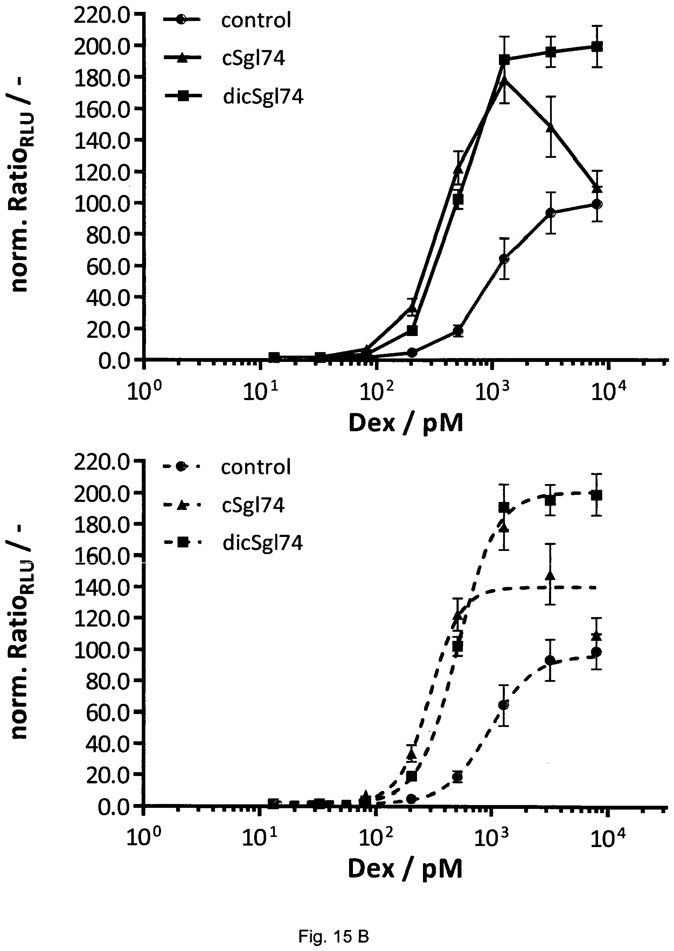

The present SNORD species can be combined in separated intronic sequences within the same gene, CDS or non-coding sequence. Nevertheless, the previously described species cSgIA and cS74 were fused to a dual SNORD species (dicSgl74) carrying a strong stem-loop-stem structure (Figure 15A). Applied to the glucocorticoid-responsive reporter cell line (GR-CALUX), the construct dicSgl74 was found to similarly increase the sensitivity, but on the other hand site led to a higher luciferase induction (Figure 15B). A tetrameric expansion of dicSgl74 including targeting human HSP90AA1 mRNA (NM_001017963.2) and human SSRP1 mRNA (NM_003146.2) resulted in SNORD XcSgl74h9ss (Figure 16A). Applying to the glucocorticoid-responsive reporter cell line (GR-CALUX), the construct XcSgl74h9ss further improved the responsiveness, sensitivity and performance compared to dual-SNORD dicSgl74 (Figure 16B). Compared to control cell line, the EC₅₀ was improved 9.8-fold applying dicSgl74 and 17.3-fold applying XcSgl74h9ss, respectively.

### Discussion:

The present invention describes for the first time the tailor-made RNA and especially mRNA stabilisation by *in silico* designed C/D box small nucleolar RNA species (SNORDs). The present invention was throughout proven using various cell lines for analytical and production purposes, using different targets and variations in SNORD structure. In addition, the combination of different SNORDs was successfully applied either by introducing multiple intronic sequences or generating a dual-SNORD (dicS species) or tetrameric SNORD (XcS species) covering multiple targets. The latter cases could increase the efficiency of the cell line in an even higher extent (Figures 15B and 16B). In all cases, the target specificity was given (e.g. Figures 6B, 9A and 14B).

The present invention relates to the following nucleotide sequences:
SEQ ID NO: 1 (5' sequence element derived from *Cricetulus griseus* Snord74): CTCGCTRTGATGW
SEQ ID NO: 2 (5' sequence element of *Cricetulus griseus* Snord74): CTCGCTATGATGA
SEQ ID NO: 3 (intermediate sequence element derived from *Cricetulus griseus* Snord74):
   CTGRCCTTGWTGATGW
SEQ ID NO: 4 (intermediate sequence element of *Cricetulus griseus* Snord74): CTGACCTTGTTGATGA
SEQ ID NO: 5 (3' sequence element derived from *Cricetulus griseus* Snord74): CTGRTGGCGG
SEQ ID NO: 6 (3' sequence element of *Cricetulus griseus* Snord74): CTGATGGCGG
SEQ ID NO: 7 (5' sequence element derived from *Cricetulus griseus* Snord78): TTGTARTGATGW
SEQ ID NO: 8 (5' sequence element of *Cricetulus griseus* Snord78): TTGTAATGATGA
SEQ ID NO: 9 (intermediate sequence element derived from *Cricetulus griseus* Snord78):
   CTGRNCTGRAAATAACATACRTGWWGW
SEQ ID NO: 10 (intermediate sequence element of *Cricetulus griseus* Snord78): CTGANCTGAAAATAACATACATGTAGA
SEQ ID NO: 11 (intermediate sequence element derived from *Cricetulus griseus* Snord78):
   CTGANCTGAAAATAACATACATGATGA
SEQ ID NO: 12 (5' specific sequence of *Cricetulus griseus* Snord74):
   AGGCTATGTTGGTAGGGACAA
SEQ ID NO: 13 (3' specific sequence of *Cricetulus griseus* Snord74):
   ATACCAACGGCT
SEQ ID NO: 14 (sequence derived from *Cricetulus griseus* Snord74): 
CTCGCTRTGATGWAGGCTATGTTGGTAGGGACAACTGRCCTTGTTGATGWATACCAACG

GCTCTGRTGGCGG
SEQ ID NO: 15 *(Cricetulus griseus* Snord74): 
CTCGCTATGATGAAGGCTATGTTGGTAGGGACAACTGACCTTGTTGATGAATACCAACG
GCTCTGATGGCGG
SEQ ID NO: 16 (sequence targeting CHO Luc7 mRNA):
   CTATTCTATGGT
SEQ ID NO: 17 (sequence targeting CHO U2 snRNA):
   ATCAGATATTAAACT
SEQ ID NO: 18 (sequence targeting CHO 28S rRNA):
   GGGTCGCCACGT
SEQ ID NO: 19 (sequence targeting CHO 18S rRNA):
   GAGTCATATTAAGCT
SEQ ID NO: 20 (sequence derived from *Cricetulus griseus* Snord78 targeting CHO Luc7 mRNA and CHO U2 snRNA): 
TTGTARTGATGWCTATTCTATGGTCTGRNCTGRAAATAACATACRTGWWGWATCAGATA
TTAAACTCTGRAGAA
SEQ ID NO: 21 (sequence derived from *Cricetulus griseus* Snord78 targeting CHO Luc7 mRNA and CHO U2 snRNA): 
TTGTAATGATGACTATTCTATGGTCTGANCTGAAAATAACATACATGATGTATCAGATA

TTAAACTCTGAAGAA
SEQ ID NO: 22 (sequence derived from *Cricetulus griseus* Snord78 targeting CHO 28S rRNA and CHO 18S rRNA): 
TTGTARTGATGWGGGTCGCCACGTCTGRNCTGRAAATAACATACRTGWWGWGAGTCATA
TTAAGCTCTGRAGAA
SEQ ID NO: 23 (sequence derived from *Cricetulus griseus* Snord78 targeting CHO 28S rRNA and CHO 18S rRNA): 
TTGTAATGATGAGGGTCGCCACGTCTGANCTGAAAATAACATACATGATGTGAGTCATA
TTAAGCTCTGAAGAA
SEQ ID NO: 24 (sequence targeting Ssrp1):
   TTAGGAGCTACAGGC
SEQ ID NO: 25 (sequence targeting Ssrp1):
   GCAGTTACTCAGATGG
SEQ ID NO: 26 (sequence derived from *Cricetulus griseus* Snord74 targeting Ssrp1): 
CTCGCTRTGATGWTTAGGAGCTACAGGCCTGRCCTTGTTGATGWGCAGTTACTCAGATG

GCTGRTGGCGG
SEQ ID NO: 27 (sequence derived from *Cricetulus griseus* Snord74 targeting Ssrp1): 
CTCGCTATGATGATTAGGAGCTACAGGCCTGACCTTGTTGATGAGCAGTTACTCAGATG
GCTGATGGCGG
SEQ ID NO: 28 (sequence targeting the SV40 polyA signal sequence):
   GTTGTGGTTTGTCCAAA
SEQ ID NO: 29 (sequence targeting the SV40 polyA signal sequence):
   TCTTATCATGTCTG
SEQ ID NO: 30 (sequence derived from *Cricetulus griseus* Snord78 targeting the SV40 polyA signal sequence): 
TTGTARTGATGWGTTGTGGTTTGTCCAAACTGRNCTGRAAATAACATACRTGWWGWTCT
TATCATGTCTGCTGRAGAA
SEQ ID NO: 31 (sequence derived from *Cricetulus griseus* Snord78 targeting the SV40 polyA signal sequence): 
TTGTAATGATGAGTTGTGGTTTGTCCAAACTGANCTGAAAATAACATACATGATGATCT

TATCATGTCTGCTGAAGAA
SEQ ID NO: 32 (sequence targeting the BGH polyA signal sequence):
   GGGGCAAACAACAGATGG
SEQ ID NO: 33 (sequence targeting the BGH polyA signal sequence):
   GAAGGCACAGTCGAGG
SEQ ID NO: 34 (sequence derived from *Cricetulus griseus* Snord78 targeting the BGH polyA signal sequence): 
TTGTARTGATGWGGGCAAACAACAGATGGCTGRNCTGRAAATAACATACRTGWWGWGA

AGGCACAGTCGAGGCTGRAGAA
SEQ ID NO: 35 (sequence derived from *Cricetulus griseus* Snord78 targeting the BGH polyA signal sequence): 
TTGTAATGATGAGGGGCAAACAACAGATGGCTGANCTGAAAATAACATACATGATGAGA
AGGCACAGTCGAGGCTGAAGAA
SEQ ID NO: 36 (3' minimal intron sequence): TACTAACTYCTCTCYCCTCYYCTCYYTTCTYYTTCTGCAG
SEQ ID NO: 37 (3' minimal intron sequence): TACTAACTCCTCTCTCCTCCTCTCCCTTCTCCTTCTGCAG
SEQ ID NO: 38 (sequence targeting BGH polyA signal):
   GGCACAGTCGAGG
SEQ ID NO: 39 (sequence derived from *Cricetulus griseus* Snord78 targeting the BGH polyA signal sequence): 
TTGTARTGATGWGGCACAGTCGAGGCTGRNCTGRAAATAACATACRTGWWGWGGGCAA
ACAACAGATGGCTGRAGAA
SEQ ID NO: 40 (sequence derived from *Cricetulus griseus* Snord78 targeting the BGH polyA signal sequence): 
TTGTAATGATGAGGCACAGTCGAGGCTGANCTGRAAATAACATACATGATGAGGGGCAA
ACAACAGATGGCTGAAGAA
SEQ ID NO: 41 (sequence targeting the recombinant hlgG 5'-UTR):
   TCGGTCCCGGTGTCTTCTA
SEQ ID NO: 42 (sequence targeting the recombinant hlgG 5'-UTR):
   TGGCGTCTCCAGGCGAT
SEQ ID NO: 43 (sequence derived from *Cricetulus griseus* Snord78 targeting the recombinant hlgG 5'-UTR): 
TTGTARTGATGWTCGGTCCCGGTGTCTTCTACTGRNCTGRAAATAACATCRTGWWGWTG
GCGTCTCCAGGCGATCTGRAGAA
SEQ ID NO: 44 (sequence derived from *Cricetulus griseus* Snord78 targeting the recombinant hlgG 5'-UTR): 
TTGTAATGATGATCGGTCCCGGTGTCTTCTACTGANCTGRAAATAACATCATGATGATG
GCGTCTCCAGGCGATCTGAAGAA
SEQ ID NO: 45 (sequence targeting human CYP1A1 mRNA):
   GTCTATGAGTTTCAGG
SEQ ID NO: 46 (sequence targeting human CYP1A1 mRNA):
   GGCCAGCCTGCTGGTCTGG
SEQ ID NO: 47 (sequence derived from *Cricetulus griseus* Snord78 targeting human CYP1A1 mRNA): 
TTGTARTGATGWGTCTATGAGTTTCAGGCTGRNCTGRAAATAACATACRTGWWGWGGCC
AGCCTGCTGGTCTGGCTGRAGAA
SEQ ID NO: 48 (sequence derived from *Cricetulus griseus* Snord78 targeting human CYP1A1 mRNA): 
TTGTAATGATGAGTCTATGAGTTTCAGGCTGANCTGRAAATAACATACATGATGAGGCC

AGCCTGCTGGTCTGGCTGAAGAA
SEQ ID NO: 49 (dual Snord construct combining cSgIA and cS74): 
CTTCTTCTTCRTGATGWGTTGTGGTTTGTCCAAACTGRNCTGRAAATAACATACRTGWW
GWTCTTATCATGTCTGCTGRAGAAGAAGCAACAACAACRTGATGWAGGCTATGTTGGTA
GGGACAACTGRCCTTGWTGATGWATACCAACGGCTCTGRTTGTTGTTG
SEQ ID NO: 50 (dual Snord construct combining cSgIA and cS74): 
CTTCTTCTTCATGATGAGTTGTGGTTTGTCCAAACTGACCTGAAAATAACATACATGAT

GATCTTATCATGTCTGCTGAAGAAGCAACAACAACATGATGAAGGCTATGTTGGTA

GGGACAACTGACCTTGTTGATGAATACCAACGGCTCTGATTGTTGTTG
SEQ ID NO: 51 (intermediate sequence element derived from *Cricetulus griseus* Snord78):
   CTGRNCTGRAAATAACATCRTGWWGW
SEQ ID NO: 52 (intermediate sequence element of *Cricetulus griseus* Snord78): CTGANCTGAAAATAACATCATGTAGA
SEQ ID NO: 53 (intermediate sequence element derived from *Cricetulus griseus* Snord78): CTGACCTGAAAATAACATCATGATGA
SEQ ID NO: 54 (tetrameric Snord XcSgl74h9ss): 
CTTCTTCTTCRTGATGWGTTGTGGTTTGTCCAAACTGRNCTGRAAATAACATACRTGWW
GWTCTTATCATGTCTGCTGRAGAAGAAGCAACAACAACRTGATGWAGGCTATGTTGGTA
GGGACAACTGRCCTTGWTGATGWATACCAACGGCTCTGRTTGTTGTTGAGGAGGAGGCR
TGATGWATTGTCAACTACAGGGACTGRAAATACCATACRTGWWGWCATCCTTGAAAATA
TATTATCTGRCCTCCTCCTACCACCACCCRTGATGWGGTAGGAGCTACAGGCCTGRNCT
GRCCTTGRTGATGWGCATGTTCAGATGGCACTGRGGTGGTGGT
SEQ ID NO: 55 (tetrameric Snord XcSgl74h9ss): 
CTTCTTCTTCATGATGAGTTGTGGTTTGTCCAAACTGATCTGAAAATAACATACATGAT
GATCTTATCATGTCTGCTGAAGAAGCAACAACAACATGATGAAGGCTATGTTGGTA
GGGACAACTGACCTTGTTGATGAATACCAACGGCTCTGATTGTTGTTGAGGAGGAGGCA
TGATGAATTGTCAACTACAGGGACTGAAAATACCATACATGATGACATCCTTGAAAATA
TATTATCTGACCTCCTCCTACCACCACCCATGATGAGGTAGGAGCTACAGGCCTGACCT
GACCTTGATGATGAGCATGTTCAGATGGCACTGAGGTGGTGGT
SEQ ID NO: 56 (intermediate sequence element derived from *Cricetulus griseus* Snord78):
   CTGRNCTGRAAATACCATACRTGWWGW
SEQ ID NO: 57 (intermediate sequence element of *Cricetulus griseus* Snord78): CTGANCTGAAAATACCATACATGTAGA
SEQ ID NO: 58 (intermediate sequence element derived from *Cricetulus griseus* Snord78):
   CTGANCTGAAAATACCATACATGATGA

## Claims

1. An isolated nucleic acid, comprising at least one of the following structures (I) and (II): 5′ SEQ ID NO: 1 Sequence A SEQ ID NO: 3 Sequence B SEQ ID NO: 5 3′ (I) 
5' SEQ ID NO: 7 | Sequence C | SEQ ID NO:
9 or 51 or 56 | Sequence D | CTGRAGAA 3' (II)
 wherein Sequence A, Sequence B, Sequence C, and Sequence D are independently nucleic acid sequences that (i) have a length of 7 to 25 base pairs (bp), and (ii) are complementary to either the same or to two different RNAs of interest.

2. The isolated nucleic acid according to claim 1, wherein in structure (I) Sequence A is SEQ ID NO: 12 and Sequence B is SEQ ID NO: 13 or *vice versa,* or
Sequence A is SEQ ID NO: 24 and Sequence B is SEQ ID NO: 25 or *vice versa.*

3. The isolated nucleic acid according to claim 1 or claim 2, wherein in structure (II)
Sequence C is SEQ ID NO: 16 and Sequence D is SEQ ID NO: 17 or *vice versa,*
Sequence C is SEQ ID NO: 18 and Sequence D is SEQ ID NO: 19 or *vice versa,*
Sequence C is SEQ ID NO: 28 and Sequence D is SEQ ID NO: 29 or *vice versa,*
Sequence C is SEQ ID NO: 32 and Sequence D is SEQ ID NO: 33 or *vice versa,*
Sequence C is SEQ ID NO: 38 and Sequence D is SEQ ID NO: 32 or *vice versa,*
Sequence C is SEQ ID NO: 41 and Sequence D is SEQ ID NO: 42 or *vice versa,* or
Sequence C is SEQ ID NO: 45 and Sequence D is SEQ ID NO: 46 or *vice versa.*

4. The isolated nucleic acid according to any one of claims 1 to 3, comprising at least two of the structures (I) and (II).

5. An isolated nucleic acid according to claim 4, wherein at least one of the nucleotide sequences according to SEQ ID NOs: 1, 5, 7, and the nucleotide sequence CTGRAGAA is at least partially replaced by a nucleotide sequence capable of forming a stem structure and/or a nucleotide sequence capable of forming a loop structure.

6. The isolated nucleic acid according to claim 5, comprising SEQ ID NO: 49 or SEQ ID NO: 54.

7. An isolated nucleic acid having at least 65% sequence identity to an isolated nucleic acid according to any one of claims 1 to 6.

8. A vector comprising at least one nucleic acid according to any one of claims 1 to 7.

9. The vector according to claim 8, wherein the vector is selected from the group consisting of plasmid vectors, viral vectors, and bacterial vectors.

10. A host cell comprising at least one nucleic acid according to any one of claims 1 to 7 and/or at least one vector according to claim 8 or claim 9.

11. The host cell according to claim 10, wherein the host cell is selected from the group consisting of CHO cells, CHO-S cells, CHO-DG44 cells, CHO-K1 cells, BHK-21 cells, PerC6 cells, CAP cells, HEK293 cells, HeLa cells, NS0 cells, SP2/0 cells, YB2/0 cells, EB66 cells, hybridoma cells, Jurkat cells, H4IIE cells, HepaRG cells, HepG2 cells, Hepa1 c1 c7 cells, U2OS cells, T47D cells, MCF-7 cells, H295R cells, LNCaP cells, amniocytes; omnipotent, pluripotent, multipotent or oligopotent mammalian stem cells; RTL-W1 cells, ZFL cells, RTG-2 cells; yeast cells derived from *Saccharomyces cerevisiae, Pichia pastoris,* or *Candida albicans;* algae cells derived from *Chlamydomonas reinhardtii, Scenedesmus obliquus,* or *Phaeodactylum tricornutum;* plant cells derived from *Arabidopsis thaliana, Nicotiana tabacum, Nicotiana rustica,* other *Nicotiana spp., Linum usitatissimum, Gossypium spp., Cannabis sativa, Zea mays, Oryza sativa, Glycine max, Lens culinaris, Triticum spp.* or *Solanum tuberosum;* filamentous fungi cells derived from *Aspergillus spp., Rhizopus spp., Trichoderma spp.* or *Penicillium spp.;* Sf9 cells, Sf21 cells, BTI-Tn-5B1-4 cells, and insect cells derived from *Spodoptera frugiperda, Bombyx mori, Mamestra brassicae, Drosophila melanogaster,* or *Trichoplusia ni;* and archaeal cells, derived from *Sulfolobus spp., Natronococcus spp., Halobacterium spp., Pyrococcus spp.,* or *Haloarcula spp..*

12. A method for protecting and/or stabilizing an RNA of interest in a host cell, comprising the step of expressing at least one nucleic acid according to any one of claims 1 to 7 in said host cell.

13. The method according to claim 12, wherein said RNA is an mRNA and protecting and/or stabilizing said mRNA results in increased expression of the protein encoded by said mRNA.

14. A method for expression of a protein of interest (POI), comprising the step of coexpressing at least one nucleic acid according to any one of claims 1 to 7 together with the coding sequence of said POI in a host cell.

15. A method of increasing the cell density (cells/ml) and/or the specific productivity (pg/cell/day) and/or the volumetric productivity (g/L/h) and/or the titer (g/L) and/or the reporter or enzyme activity of a protein of interest (POI) in a culture of host cells, comprising the step of expressing at least one nucleic acid according to any one of claims 1 to 7 in said host cells.


SEQUENCE LISTING

<110>  RWTH Aachen

<120>  RNA Toolbox

<130>  R 2753EU - kl

<160>  58

<170>  PatentIn version 3.5

<210>  1
<211>  13
<212>  DNA
<213>  Artificial sequence

<220>
<223>  sequence derived from Cricetulus griseus Snord 74

<400>  1
ctcgctrtga tgw                                                          13


<210>  2
<211>  13
<212>  DNA
<213>  Cricetulus griseus

<400>  2
ctcgctatga tga                                                          13


<210>  3
<211>  16
<212>  DNA
<213>  Artificial sequence

<220>
<223>  sequence derived from Cricetulus griseus Snord 74

<400>  3
ctgrccttgw tgatgw                                                       16


<210>  4
<211>  16
<212>  DNA
<213>  Cricetulus griseus

<400>  4
ctgaccttgt tgatga                                                       16


<210>  5
<211>  10
<212>  DNA
<213>  Artificial sequence

<220>
<223>  sequence derived from Cricetulus griseus Snord 74

<400>  5

ctgrtggcgg                                                              10


<210>  6
<211>  10
<212>  DNA
<213>  Cricetulus griseus

<400>  6
ctgatggcgg                                                              10


<210>  7
<211>  12
<212>  DNA
<213>  Artificial sequence

<220>
<223>  sequence derived from Cricetulus griseus Snord 78

<400>  7
ttgtartgat gw                                                           12


<210>  8
<211>  12
<212>  DNA
<213>  Cricetulus griseus

<400>  8
ttgtaatgat ga                                                           12


<210>  9
<211>  27
<212>  DNA
<213>  Artificial sequence

<220>
<223>  sequence derived from Cricetulus griseus Snord 78


<220>
<221>  misc_feature
<222>  (5)..(5)
<223>  n is a, c, g, or t

<400>  9
ctgrnctgra aataacatac rtgwwgw                                           27


<210>  10
<211>  27
<212>  DNA
<213>  Artificial sequence

<220>
<223>  adapted sequence derived from a part of the Cricetulus griseus
       Snord 78


<220>
<221>  misc_feature

<222>  (5)..(5)
<223>  n is a, c, g, or t

<400>  10
ctganctgaa aataacatac atgtaga                                          27


<210>  11
<211>  27
<212>  DNA
<213>  Artificial sequence

<220>
<223>  adapted sequence derived from a part of the Cricetulus griseus
       Snord 78


<220>
<221>  misc_feature
<222>  (5)..(5)
<223>  n is a, c, g, or t

<400>  11
ctganctgaa aataacatac atgatga                                          27


<210>  12
<211>  21
<212>  DNA
<213>  Cricetulus griseus

<400>  12
aggctatgtt ggtagggaca a                                                21


<210>  13
<211>  12
<212>  DNA
<213>  Cricetulus griseus

<400>  13
ataccaacgg ct                                                          12


<210>  14
<211>  72
<212>  DNA
<213>  Artificial sequence

<220>
<223>  sequence derived from Cricetulus griseus Snord 74

<400>  14
ctcgctrtga tgwaggctat gttggtaggg acaactgrcc ttgttgatgw ataccaacgg      60

ctctgrtggc gg                                                          72


<210>  15
<211>  72
<212>  DNA
<213>  Cricetulus griseus

<400>  15
ctcgctatga tgaaggctat gttggtaggg acaactgacc ttgttgatga ataccaacgg      60

ctctgatggc gg                                                          72


<210>  16
<211>  12
<212>  DNA
<213>  Artificial sequence

<220>
<223>  sequence targeting CHO Luc7 mRNA

<400>  16
ctattctatg gt                                                          12


<210>  17
<211>  15
<212>  DNA
<213>  Artificial sequence

<220>
<223>  sequence targeting CHO U2 snRNA

<400>  17
atcagatatt aaact                                                       15


<210>  18
<211>  12
<212>  DNA
<213>  Artificial sequence

<220>
<223>  sequence targeting CHO 28S rRNA

<400>  18
gggtcgccac gt                                                          12


<210>  19
<211>  15
<212>  DNA
<213>  Artificial sequence

<220>
<223>  sequence targeting CHO 18S rRNA

<400>  19
gagtcatatt aagct                                                       15


<210>  20
<211>  74
<212>  DNA
<213>  Artificial sequence

<220>
<223>  sequence derived from Cricetulus griseus Snord 78 targeting CHO
       Luc7 mRNA and CHO U2 snRNA

<220>
<221>  misc_feature
<222>  (29)..(29)
<223>  n is a, c, g, or t

<400>  20
ttgtartgat gwctattcta tggtctgrnc tgraaataac atacrtgwwg watcagatat      60

taaactctgr agaa                                                        74


<210>  21
<211>  74
<212>  DNA
<213>  Artificial sequence

<220>
<223>  sequence derived from Cricetulus griseus Snord78 targeting CHO
      Luc7 mRNA and CHO U2 snRNA


<220>
<221>  misc_feature
<222>  (29)..(29)
<223>  n is a, c, g, or t

<400>  21
ttgtaatgat gactattcta tggtctganc tgaaaataac atacatgatg tatcagatat      60

taaactctga agaa                                                        74


<210>  22
<211>  74
<212>  DNA
<213>  Artificial sequence

<220>
<223>  sequence derived from Cricetulus griseus Snord 78 targeting CHO
      28S rRNA and CHO 18S rRNA


<220>
<221>  misc_feature
<222>  (29)..(29)
<223>  n is a, c, g, or t

<400>  22
ttgtartgat gwgggtcgcc acgtctgrnc tgraaataac atacrtgwwg wgagtcatat      60

taagctctgr agaa                                                        74


<210>  23
<211>  74
<212>  DNA
<213>  Artificial sequence

<220>
<223>  sequence derived from Cricetulus griseus Snord78 targeting CHO
      28S rRNA and CHO 18S rRNA

<220>
<221>  misc_feature
<222>  (29)..(29)
<223>  n is a, c, g, or t

<400>  23
ttgtaatgat gagggtcgcc acgtctganc tgaaaataac atacatgatg tgagtcatat      60

taagctctga agaa                                                        74


<210>  24
<211>  15
<212>  DNA
<213>  Artificial sequence

<220>
<223>  sequence targeting human Ssrp1

<400>  24
ttaggagcta caggc                                                       15


<210>  25
<211>  16
<212>  DNA
<213>  Artificial sequence

<220>
<223>  sequence targeting human Ssrp1

<400>  25
gcagttactc agatgg                                                      16


<210>  26
<211>  70
<212>  DNA
<213>  Artificial sequence

<220>
<223>  sequence derived from Cricetulus griseus Snord74 targeting Ssrp1

<400>  26
ctcgctrtga tgwttaggag ctacaggcct grccttgttg atgwgcagtt actcagatgg      60

ctgrtggcgg                                                             70


<210>  27
<211>  70
<212>  DNA
<213>  Artificial sequence

<220>
<223>  sequence derived from Cricetulus griseus Snord74 targeting Ssrp1

<400>  27
ctcgctatga tgattaggag ctacaggcct gaccttgttg atgagcagtt actcagatgg      60

ctgatggcgg                                                             70

<210>  28
<211>  17
<212>  DNA
<213>  Artificial sequence

<220>
<223>  sequence targeting the SV40 polyA signal sequence

<400>  28
gttgtggttt gtccaaa                                                    17


<210>  29
<211>  14
<212>  DNA
<213>  Artificial sequence

<220>
<223>  sequence targeting the SV40 polyA signal sequence

<400>  29
tcttatcatg tctg                                                       14


<210>  30
<211>  78
<212>  DNA
<213>  Artificial sequence

<220>
<223>  sequence derived from Cricetulus griseus Snord78 targeting the
       SV40 polyA signal sequence


<220>
<221>  misc_feature
<222>  (34)..(34)
<223>  n is a, c, g, or t

<400>  30
ttgtartgat gwgttgtggt ttgtccaaac tgrnctgraa ataacatacr tgwwgwtctt      60

atcatgtctg ctgragaa                                                   78


<210>  31
<211>  78
<212>  DNA
<213>  Artificial sequence

<220>
<223>  sequence derived from Cricetulus griseus Snord78 targeting the
       SV40 polyA signal sequence


<220>
<221>  misc_feature
<222>  (34)..(34)
<223>  n is a, c, g, or t

<400>  31
ttgtaatgat gagttgtggt ttgtccaaac tganctgaaa ataacataca tgatgatctt      60

atcatgtctg ctgaagaa                                                    78

<210>  32
<211>  18
<212>  DNA
<213>  Artificial sequence

<220>
<223>  sequence targeting the BGH polyA signal sequence

<400>  32
ggggcaaaca acagatgg                                                    18

<210>  33
<211>  16
<212>  DNA
<213>  Artificial sequence

<220>
<223>  sequence targeting the BGH polyA signal sequence

<400>  33
gaaggcacag tcgagg                                                      16

<210>  34
<211>  81
<212>  DNA
<213>  Artificial sequence

<220>
<223>  sequence derived from Cricetulus griseus Snord78 targeting the
       BGH polyA signal sequence

<220>
<221>  misc_feature
<222>  (35)..(35)
<223>  n is a, c, g, or t

<400>  34
ttgtartgat gwggggcaaa caacagatgg ctgrnctgra aataacatac rtgwwgwgaa      60

ggcacagtcg aggctgraga a                                                81

<210>  35
<211>  81
<212>  DNA
<213>  Artificial sequence

<220>
<223>  sequence derived from Cricetulus griseus Snord78 targeting the
       BGH polyA signal sequence

<220>
<221>  misc_feature
<222>  (35)..(35)
<223>  n is a, c, g, or t

<400>  35
ttgtaatgat gaggggcaaa caacagatgg ctganctgaa aataacatac atgatgagaa      60

ggcacagtcg aggctgaaga a                                                81


<210>  36
<211>  40
<212>  DNA
<213>  Artificial sequence

<220>
<223>  5′ minimal intron sequence

<400>  36
tactaactyc tctcycctcy yctcyyttct yyttctgcag                            40


<210>  37
<211>  40
<212>  DNA
<213>  Artificial sequence

<220>
<223>  3′ minimal intron sequence

<400>  37
tactaactcc tctctcctcc tctcccttct ccttctgcag                            40


<210>  38
<211>  13
<212>  DNA
<213>  Artificial sequence

<220>
<223>  sequence targeting BGH polyA signal

<400>  38
ggcacagtcg agg                                                         13


<210>  39
<211>  78
<212>  DNA
<213>  Artificial sequence

<220>
<223>  sequence derived from Cricetulus griseus Snord78 targeting the
       BGH polyA signal sequence


<220>
<221>  misc_feature
<223>  n is a, c, g, or t

<220>
<221>  misc_feature
<222>  (30)..(30)
<223>  n is a, c, g, or t

<400>  39

ttgtartgat gwggcacagt cgaggctgrn ctgraaataa catacrtgww gwggggcaaa 60

caacagatgg ctgragaa 78

<210> 40
<211> 78
<212> DNA
<213> Artificial sequence

<220>
<223> sequence derived from Cricetulus griseus Snord78 targeting the
      BGH polyA signal sequence

<220>
<221> misc_feature
<222> (30)..(30)
<223> n is a, c, g, or t

<400> 40
ttgtaatgat gaggcacagt cgaggctgan ctgraaataa catacatgat gaggggcaaa 60

caacagatgg ctgaagaa 78

<210> 41
<211> 19
<212> DNA
<213> Artificial sequence

<220>
<223> sequence targeting the hIgG 5'-UTR

<400> 41
tcggtcccgg tgtcttcta 19

<210> 42
<211> 17
<212> DNA
<213> Artificial sequence

<220>
<223> sequence targeting the hIgG 5'-UTR

<400> 42
tggcgtctcc aggcgat 17

<210> 43
<211> 82
<212> DNA
<213> Artificial sequence

<220>
<223> sequence derived from Cricetulus griseus Snord78 targeting the
      hIgG 5'-UTR

<220>
<221> misc_feature
<222> (36)..(36)

<223>  n is a, c, g, or t

<400>  43
ttgtartgat gwtcggtccc ggtgtcttct actgrnctgr aaataacatc rtgwwgwtgg      60

cgtctccagg cgatctgrag aa                                              82


<210>  44
<211>  82
<212>  DNA
<213>  Artificial sequence

<220>
<223>  sequence derived from Cricetulus griseus Snord78 targeting the
       hIgG 5'-UTR


<220>
<221>  misc_feature
<222>  (36)..(36)
<223>  n is a, c, g, or t

<400>  44
ttgtaatgat gatcggtccc ggtgtcttct actganctgr aaataacatc atgatgatgg      60

cgtctccagg cgatctgaag aa                                              82


<210>  45
<211>  14
<212>  DNA
<213>  Artificial sequence

<220>
<223>  sequence targeting human CYP1A1 mRNA

<400>  45
gtctatgagt ttca                                                       14


<210>  46
<211>  19
<212>  DNA
<213>  Artificial sequence

<220>
<223>  sequence targeting human CYP1A1 mRNA

<400>  46
ggccagcctg ctggtctgg                                                  19


<210>  47
<211>  82
<212>  DNA
<213>  Artificial sequence

<220>
<223>  sequence derived from Cricetulus griseus Snord78 targeting human
       CYP1A1 mRNA

<220>
<221>  misc_feature
<222>  (33)..(33)
<223>  n is a, c, g, or t

<400>  47
ttgtartgat gwgtctatga gtttcaggct grnctgraaa taacatacrt gwwgwggcca      60

gcctgctggt ctggctgrag aa                                              82


<210>  48
<211>  82
<212>  DNA
<213>  Artificial sequence

<220>
<223>  sequence derived from Cricetulus griseus Snord78 targeting human
       CYP1A1 mRNA


<220>
<221>  misc_feature
<222>  (33)..(33)
<223>  n is a, c, g, or t

<400>  48
ttgtaatgat gagtctatga gtttcaggct ganctgraaa taacatacat gatgaggcca      60

gcctgctggt ctggctgaag aa                                              82


<210>  49
<211>  166
<212>  DNA
<213>  Artificial sequence

<220>
<223>  dual Snord construct combining cSglA and cS74


<220>
<221>  misc_feature
<222>  (39)..(39)
<223>  n is a, c, g, or t

<400>  49
cttcttcttc rtgatgwgtt gtggtttgtc caaactgrnc tgraaataac atacrtgwwg      60

wtcttatcat gtctgctgra gaagaagcaa caacaacrtg atgwaggcta tgttggtagg     120

gacaactgrc cttgwtgatg wataccaacg gctctgrttg ttgttg                    166


<210>  50
<211>  166
<212>  DNA
<213>  Artificial sequence

<220>
<223>  dual Snord construct combining cSglA and cS74

<400>  50

cttcttcttc atgatgagtt gtggtttgtc caaactgacc tgaaaataac atacatgatg 60

atcttatcat gtctgctgaa gaagaagcaa caacaacatg atgaaggcta tgttggtagg 120

gacaactgac cttgttgatg aataccaacg gctctgattg ttgttg 166

<210> 51
<211> 26
<212> DNA
<213> Artificial sequence

<220>
<223> sequence derived from Cricetulus griseus Snord 78

<220>
<221> misc_feature
<222> (5)..(5)
<223> n is a, c, g, or t

<400> 51
ctgrnctgra aataacatcr tgwwgw 26

<210> 52
<211> 26
<212> DNA
<213> Artificial sequence

<220>
<223> adapted sequence derived from a part of the Cricetulus griseus
      Snord 78

<220>
<221> misc_feature
<222> (5)..(5)
<223> n is a, c, g, or t

<400> 52
ctganctgaa aataacatca tgtaga 26

<210> 53
<211> 26
<212> DNA
<213> Artificial sequence

<220>
<223> adapted sequence derived from a part of the Cricetulus griseus
      Snord 78

<400> 53
ctgacctgaa aataacatca tgatga 26

<210> 54
<211> 338
<212> DNA
<213> Artificial sequence

<220>

<223>  tetrameric Snord XcSgl74h9ss

<220>
<221>  misc_feature
<222>  (39)..(39)
<223>  n is a, c, g, or t

<220>
<221>  misc_feature
<222>  (293)..(293)
<223>  n is a, c, g, or t

<400>  54
cttcttcttc rtgatgwgtt gtggtttgtc caaactgrnc tgraaataac atacrtgwwg      60

wtcttatcat gtctgctgra gaagaagcaa caacaacrtg atgwaggcta tgttggtagg      120

gacaactgrc cttgwtgatg wataccaacg gctctgrttg ttgttgagga ggaggcrtga      180

tgwattgtca actacaggga ctgraaatac catacrtgww gwcatccttg aaaatatatt      240

atctgrcctc ctcctaccac cacccrtgat gwggtaggag ctacaggcct grnctgrcct      300

tgrtgatgwg catgttcaga tggcactgrg gtggtggt                              338

<210>  55
<211>  338
<212>  DNA
<213>  Artificial sequence

<220>
<223>  tetrameric Snord XcSgl74h9ss

<400>  55
cttcttcttc atgatgagtt gtggtttgtc caaactgatc tgaaaataac atacatgatg      60

atcttatcat gtctgctgaa gaagaagcaa caacaacatg atgaaggcta tgttggtagg      120

gacaactgac cttgttgatg aataccaacg gctctgattg ttgttgagga ggaggcatga      180

tgaattgtca actacaggga ctgaaaatac catacatgat gacatccttg aaaatatatt      240

atctgacctc ctcctaccac cacccatgat gaggtaggag ctacaggcct gacctgacct      300

tgatgatgag catgttcaga tggcactgag gtggtggt                              338

<210>  56
<211>  27
<212>  DNA
<213>  Artificial sequence

<220>
<223>  sequence derived from Cricetulus griseus Snord 78

<220>
<221>  misc_feature
<222>  (5)..(5)
<223>  n is a, c, g, or t

<400>  56
ctgrnctgra aataccatac rtgwwgw                                          27


<210>  57
<211>  27
<212>  DNA
<213>  Artificial sequence

<220>
<223>  adapted sequence derived from a part of the Cricetulus griseus
       Snord 78


<220>
<221>  misc_feature
<222>  (5)..(5)
<223>  n is a, c, g, or t

<400>  57
ctganctgaa aataccatac atgtaga                                          27


<210>  58
<211>  27
<212>  DNA
<213>  Artificial sequence

<220>
<223>  adapted sequence derived from a part of the Cricetulus griseus
       Snord 78


<220>
<221>  misc_feature
<222>  (5)..(5)
<223>  n is a, c, g, or t

<400>  58
ctganctgaa aataccatac atgatga                                          27